# EUROPEAN PATENT APPLICATION

(11) **EP 3 950 766 A1**
(43) Date of publication of application: **09.02.2022**
(21) Application number: 20784732.8
(22) Date of filing: 16.03.2020
(51) Int. Cl.: C08G 61/10, C09K 11/06, H01L 51/50

(54) **LUMINESCENT ELEMENT AND COMPOSITION FOR LUMINESCENT ELEMENT**

(30) Priority: 29.03.2019 JP 2019066198
(71) Applicant: SUMITOMO CHEMICAL COMPANY LIMITED, Chuo-ku Tokyo 104-8260 (JP)
(72) Inventor: SASADA, Toshiaki, Tsukuba-shi, Ibaraki 300-3294 (JP); MATSUMOTO, Ryuji, Ishikawa 923-1201 (JP)
(74) Representative: J A Kemp LLP
(86) International application number: PCT/JP2020/011385
(87) International publication number: WO 2020/203211

(57) **Abstract**

To provide a composition which is useful for producing a light emitting device excellent in external quantum efficiency, and to provide a light emitting device containing the composition.

A light emitting device comprising an anode, a cathode, and an organic layer disposed between the anode and the cathode and containing a composition for light emitting device, wherein the composition for light emitting device contains at least two compounds (A) selected from the group consisting of a compound represented by the formula (FH) and a polymer compound containing a constitutional unit having a group obtained by removing from a compound represented by the formula (FH) one or more hydrogen atoms, and a compound (B) having a condensed hetero ring skeleton (b) containing a boron atom and a nitrogen atom in the ring.

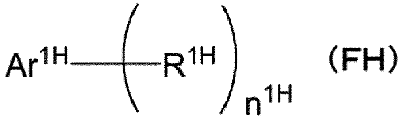

## Description

### Technical Field

The present invention relates to a light emitting device and a composition for light emitting device.

### Background Art

Light emitting devices such as an organic electroluminescent device and the like can be suitably used, for example, for display and illumination. As the light emitting material used for a light emitting layer of a light emitting device, for example, Patent Document 1 suggests a composition containing a compound G1.

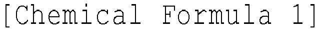

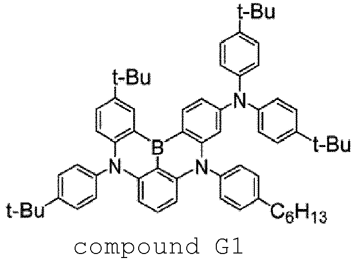

### [Prior Art Document]

### [Patent Document]

[Patent Document 1] International Publication WO2018/062278

### Summary of the Invention

### Problem to be Solved by the Invention

However, a light emitting device fabricated using the above-described composition was not necessarily sufficient in external quantum efficiency.

Then, the present invention has an object of providing a composition which is useful for producing a light emitting device excellent in external quantum efficiency, and a light emitting device containing the composition.

### Means for Solving the Problem

The present inventors have intensively studied to solve the above-described problem and resultantly found that a light emitting device excellent in external quantum efficiency is formed by a composition for light emitting device containing two or more specific compounds (A) and a specific compound (B), leading to completion of the present invention.

That is, the present invention provides the following [1] to [13].
[1] A light emitting device comprising
   an anode,
   a cathode, and
   an organic layer disposed between the above-described anode and the above-described cathode and containing a composition for light emitting device, wherein
   the above-described composition for light emitting device contains
      at least two compounds (A) selected from the group consisting of a compound represented by the formula (FH) and a polymer compound containing a constitutional unit having a group obtained by removing from a compound represented by the formula (FH) one or more hydrogen atoms, and
      a compound (B) having a condensed hetero ring skeleton (b) containing a boron atom and a nitrogen atom in the ring: 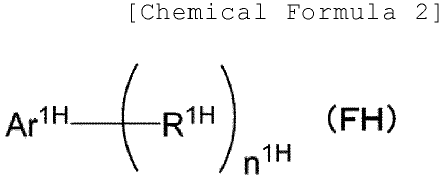 [wherein,
         n^{1H} represents an integer of 0 or more.
         Ar^{1H} represents a group obtained by removing from a heterocyclic compound containing a nitrogen atom in the ring n^{1H} or more hydrogen atoms bonding directly to atoms constituting the ring, and this group optionally has a substituent. When a plurality of the substituents are present, they may be the same or different and may be combined together to form a ring together with atoms to which they are attached. The above-described heterocyclic compound is a heterocyclic compound not containing the above-described condensed hetero ring skeleton (b).
         R^{1H} represents an aryl group, a monovalent hetero ring group or a substituted amino group, and these groups optionally have a substituent. When a plurality of the substituents are present, they may be the same or different and may be combined together to form a ring together with atoms to which they are attached. When a plurality of R^{1H} are present, they may be the same or different and may be combined together to form a ring together with atoms to which they are attached. The above-described monovalent hetero ring group is a group obtained by removing from a heterocyclic compound not containing the above-described condensed hetero ring skeleton (b) one hydrogen atom bonding directly to an atom constituting the ring, and this group optionally has a substituent.
   The substituent which Ar^{1H} optionally has and R^{1H} may be combined together to form a ring together with atoms to which they are attached.].
[2] The light emitting device according to [1], wherein at least one of the above-described polymer compound is contained as the above-described compound (A) .
[3] The light emitting device according to [1] or [2], wherein the above-described heterocyclic compound in the above-described Ar^{1H} is a monocyclic heterocyclic compound containing a nitrogen atom in the ring, a dicyclic heterocyclic compound containing a nitrogen atom in the ring or a tricyclic heterocyclic compound containing a nitrogen atom in the ring.
[4] The light emitting device according to [3], wherein the above-described heterocyclic compound in the above-described Ar^{1H} is oxadiazole, thiadiazole, pyrrole, diazole, triazole, pyridine, diazabenzene, triazine, azanaphthalene, diazanaphthalene, indole, benzodiazole, carbazole, azacarbazole, diazacarbazole, phenoxazine, phenothiazine, 9,10-dihydroacridine, 5,10-dihydrophenazine, azaanthracene, diazaanthracene, azaphenanthrene or diazaphenanthrene.
[5] The light emitting device according to any one of [1] to [4], wherein the above-described compound (B) is a compound represented by the formula (1-1), a compound represented by the formula (1-2) or a compound represented by the formula (1-3): 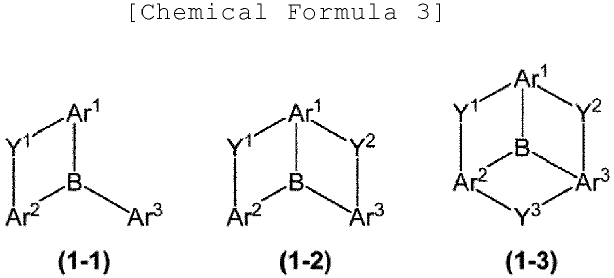 [wherein,
   Ar¹, Ar² and Ar³ each independently represent an aromatic hydrocarbon group or a hetero ring group, and these groups optionally have a substituent. When a plurality of the substituents are present, they may be the same or different and may be combined together to form a ring together with atoms to which they are attached.
   Y¹ represents a group represented by -N(Ry)-.
   Y² and Y³ each independently represent a single bond, an oxygen atom, a sulfur atom, a selenium atom, a group represented by -N(Ry)-, an alkylene group or a cycloalkylene group, and these groups optionally have a substituent. When a plurality of the substituents are present, they may be the same or different and may be combined together to form a ring together with atoms to which they are attached. Ry represents a hydrogen atom, an alkyl group, a cycloalkyl group, an aryl group or a monovalent hetero ring group, and these groups optionally have a substituent. When a plurality of the substituents are present, they may be the same or different and may be combined together to form a ring together with atoms to which they are attached. When a plurality of Ry are present, they may be the same or different. Ry may be bonded directly or via a connecting group to Ar¹, Ar² or Ar³.].
[6] The light emitting device according to [5], wherein the above-described Y² and the above-described Y³ are each a group represented by -N(Ry)-.
[7] The light emitting device according to any one of [1] to [6], wherein the above-described composition for light emitting device further contains at least one selected from the group consisting of a hole transporting material, a hole injection material, an electron transporting material, an electron injection material, a light emitting material, an antioxidant and a solvent.
[8] A composition for light emitting device comprising
   at least two compounds (A) selected from the group consisting of a compound represented by the formula (FH) and a polymer compound containing a constitutional unit having a group obtained by removing from a compound represented by the formula (FH) one or more hydrogen atoms, and
   a compound (B) having a condensed hetero ring skeleton (b) containing a boron atom and a nitrogen atom in the ring: 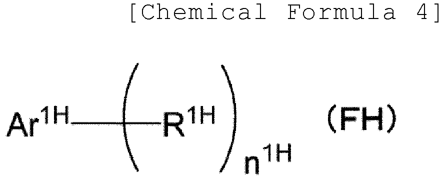 [wherein,
      n^{1H} represents an integer of 0 or more.
      Ar^{1H} represents a group obtained by removing from a heterocyclic compound containing a nitrogen atom in the ring n^{1H} or more hydrogen atoms bonding directly to atoms constituting the ring, and this group optionally has a substituent. When a plurality of the substituents are present, they may be the same or different and may be combined together to form a ring together with atoms to which they are attached. The above-described heterocyclic compound is a heterocyclic compound not containing the above-described condensed hetero ring skeleton (b).
      R^{1H} represents an aryl group, a monovalent hetero ring group or a substituted amino group, and these groups optionally have a substituent. When a plurality of the substituents are present, they may be the same or different and may be combined together to form a ring together with atoms to which they are attached. When a plurality of R^{1H} are present, they may be the same or different and may be combined together to form a ring together with atoms to which they are attached. The above-described monovalent hetero ring group is a group obtained by removing from a heterocyclic compound not containing the above-described condensed hetero ring skeleton (b) one hydrogen atom bonding directly to an atom constituting the ring, and this group optionally has a substituent.
   The substituent which Ar^{1H} optionally has and R^{1H} may be combined together to form a ring together with atoms to which they are attached.].
[9] The composition for light emitting device according to [8], wherein at least one of the above-described polymer compound is contained as the above-described compound (A).
[10] The composition for light emitting device according to [8] or [9], wherein the above-described heterocyclic compound in the above-described Ar^{1H} is a monocyclic heterocyclic compound containing a nitrogen atom in the ring, a dicyclic heterocyclic compound containing a nitrogen atom in the ring or a tricyclic heterocyclic compound containing a nitrogen atom in the ring.
[11] The composition for light emitting device according to any one of [8] to [10], wherein the above-described compound (B) is a compound represented by the formula (1-1), a compound represented by the formula (1-2) or a compound represented by the formula (1-3): 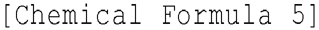 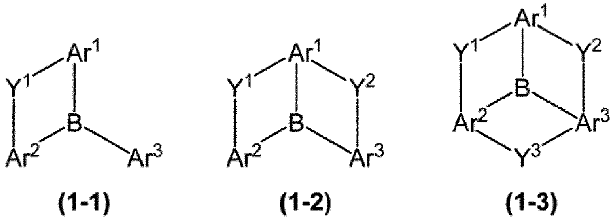 [wherein,
   Ar¹, Ar² and Ar³ each independently represent an aromatic hydrocarbon group or a hetero ring group, and these groups optionally have a substituent. When a plurality of the substituents are present, they may be the same or different and may be combined together to form a ring together with atoms to which they are attached.
   Y¹ represents a group represented by -N(Ry)-.
   Y² and Y³ each independently represent a single bond, an oxygen atom, a sulfur atom, a selenium atom, a group represented by -N(Ry)-, an alkylene group or a cycloalkylene group, and these groups optionally have a substituent. When a plurality of the substituents are present, they may be the same or different and may be combined together to form a ring together with atoms to which they are attached. Ry represents a hydrogen atom, an alkyl group, a cycloalkyl group, an aryl group or a monovalent hetero ring group, and these groups optionally have a substituent. When a plurality of the substituents are present, they may be the same or different and may be combined together to form a ring together with atoms to which they are attached. When a plurality of Ry are present, they may be the same or different. Ry may be bonded directly or via a connecting group to Ar¹, Ar² or Ar³.].
[12] The composition for light emitting device according to [11], wherein the above-described Y² and the above-described Y³ are each a group represented by -N(Ry)-.
[13] The composition for light emitting device according to any one of [8] to [12], further comprising at least one selected from the group consisting of a hole transporting material, a hole injection material, an electron transporting material, an electron injection material, a light emitting material, an antioxidant and a solvent.

### Effect of the Invention

According to the present invention, it is possible to provide a composition which is useful for producing a light emitting device excellent in external quantum efficiency. Further, according to the present invention, it is possible to provide a light emitting device containing the composition.

### Modes for Carrying Out the Invention

Suitable embodiments of the present invention will be illustrated in detail below.

### <Explanation of common terms>

Terms commonly used in the present specification have the following meanings unless otherwise stated.

"Room temperature" denotes 25°C.

Me represents a methyl group, Et represents an ethyl group, Bu represents a butyl group, i-Pr represents an isopropyl group, and t-Bu represents a tert-butyl group.

A hydrogen atom may be a heavy hydrogen atom or a light hydrogen atom.

"The low molecular weight compound" means a compound having no molecular weight distribution and having a molecular weight of 1×10⁴ or less.

"The polymer compound" means a polymer having molecular weight distribution and having a polystyrene-equivalent number-average molecular weight of 1×10³ or more (for example, 1×10³ to 1×10⁸).

"The constitutional unit" means a unit occurring once or more times in the polymer compound.

The polymer compound may be any of a block copolymer, a random copolymer, an alternating copolymer and a graft copolymer, and may also be another form.

The end group of the polymer compound is preferably a stable group since if a polymerization active group remains intact there, there is a possibility of a decrease in a light emitting property or luminance life when the polymer compound is used for fabrication of a light emitting device. The end group of the polymer compound is preferably a group conjugatively bonded to the main chain and includes, for example, groups bonding to an aryl group or a monovalent hetero ring group linking to the main chain of the polymer compound via a carbon-carbon bond.

"The alkyl group" may be any of linear or branched. The number of carbon atoms of the linear alkyl group, not including the number of carbon atoms of the substituent, is usually 1 to 50, preferably 1 to 20, and more preferably 1 to 10. The number of carbon atoms of the branched alkyl group, not including the number of carbon atoms of the substituent, is usually 3 to 50, preferably 3 to 20, and more preferably 4 to 10.

The alkyl group optionally has a substituent. The alkyl group includes, for example, a methyl group, an ethyl group, a propyl group, an isopropyl group, a butyl group, a 2-butyl group, an isobutyl group, a tert-butyl group, a pentyl group, an isoamyl group, a 2-ethylbutyl group, a hexyl group, a heptyl group, an octyl group, a 2-ethylhexyl group, a 3-propylheptyl group, a decyl group, a 3,7-dimethyloctyl group, a 2-ethyloctyl group, a 2-hexyldecyl group and a dodecyl group. Further, the alkyl group may also be a group obtained by substituting a part or all of hydrogen atoms in these groups with a cycloalkyl group, an alkoxy group, a cycloalkoxy group, an aryl group, a fluorine atom or the like. Such an alkyl group includes, for example, a trifluoromethyl group, a pentafluoroethyl group, a perfluorobutyl group, a perfluorohexyl group, a perfluorooctyl group, a 3-phenylpropyl group, a 3-(4-methylphenyl)propyl group, a 3-(3,5-di-hexylphenyl)propyl group and a 6-ethyloxyhexyl group.

The number of carbon atoms of "the cycloalkyl group", not including the number of carbon atoms of the substituent, is usually 3 to 50, and preferably 4 to 10. The cycloalkyl group optionally has a substituent. The cycloalkyl group includes, for example, a cyclohexyl group and a methylcyclohexyl group.

The number of carbon atoms of "the alkylene group", not including the number of carbon atoms of the substituent, is usually 1 to 20, preferably 1 to 15, and more preferably 1 to 10. The alkylene group optionally has a substituent. The alkylene group includes, for example, a methylene group, an ethylene group, a propylene group, a butylene group, a hexylene group and an octylene group.

The number of carbon atoms of "the cycloalkylene group", not including the number of carbon atoms of the substituent, is usually 3 to 20, and preferably 4 to 10. The cycloalkylene group optionally has a substituent. The cycloalkylene group includes, for example, a cyclohexylene group.

"The aromatic hydrocarbon group" means a group obtained by removing from an aromatic hydrocarbon one or more hydrogen atoms bonding directly to atoms constituting the ring. The group obtained by removing from an aromatic hydrocarbon one hydrogen atom bonding directly to an atom constituting the ring is referred to also as "aryl group". The group obtained by removing from an aromatic hydrocarbon two hydrogen atoms bonding directly to atoms constituting the ring is referred to also as "arylene group".

The number of carbon atoms of the aromatic hydrocarbon group, not including the number of carbon atoms of the substituent, is usually 6 to 60, preferably 6 to 40, and more preferably 6 to 20.

"The aromatic hydrocarbon group" includes, for example, groups obtained by removing from a monocyclic aromatic hydrocarbon (including, for example, benzene) or a polycyclic aromatic hydrocarbon (including, for example, dicyclic aromatic hydrocarbons such as naphthalene, indene and the like; tricyclic aromatic hydrocarbons such as anthracene, phenanthrene, dihydrophenanthrene, fluorene and the like; tetracyclic aromatic hydrocarbons such as benzoanthracene, benzophenanthrene, benzofluorene, pyrene, fluoranthene and the like; pentacyclic aromatic hydrocarbons such as dibenzoanthracene, dibenzophenanthrene, dibenzofluorene, perylene, benzofluoranthene and the like; hexacyclic aromatic hydrocarbons such as spirobifluorene and the like; and, heptacyclic aromatic hydrocarbons such as benzospirobifluorene, acenaphthofluoranthene and the like) one or more hydrogen atoms bonding directly to atoms constituting the ring. The aromatic hydrocarbon group includes groups obtained by bonding a plurality of these groups. The aromatic hydrocarbon group optionally has a substituent.

"The alkoxy group" may be any of linear or branched. The number of carbon atoms of the linear alkoxy group, not including the number of carbon atoms of the substituent, is usually 1 to 40, and preferably 1 to 10. The number of carbon atoms of the branched alkoxy group, not including the number of carbon atoms of the substituent, is usually 3 to 40, and preferably 4 to 10.

The alkoxy group optionally has a substituent. The alkoxy group includes, for example, a methoxy group, an ethoxy group, an isopropyloxy group, a butyloxy group, a hexyloxy group, a 2-ethylhexyloxy group, a 3,7-dimethyloctyloxy group and a lauryloxy group.

The number of carbon atoms of "the cycloalkoxy group", not including the number of carbon atoms of the substituent, is usually 3 to 40, and preferably 4 to 10. The cycloalkoxy group optionally has a substituent. The cycloalkoxy group includes, for example, a cyclohexyloxy group.

The number of carbon atoms of "the aryloxy group", not including the number of carbon atoms of the substituent, is usually 6 to 60, preferably 6 to 40, and more preferably 6 to 20. The aryloxy group optionally has a substituent. The aryloxy group includes, for example, a phenoxy group, a naphthyloxy group, an anthracenyloxy group and a pyrenyloxy group.

"The hetero ring group" means a group obtained by removing from a heterocyclic compound one or more hydrogen atoms bonding directly to atoms constituting the ring. Of the hetero ring groups, "an aromatic hetero ring group" which is a group obtained by removing from an aromatic heterocyclic compound one or more hydrogen atoms bonding directly to atoms constituting the ring is preferable. The group obtained by removing from a heterocyclic compound p hydrogen atoms bonding directly to atoms constituting the ring (p represents an integer of 1 or more) is referred to also as "p-valent hetero ring group". The group obtained by removing from an aromatic heterocyclic compound p hydrogen atoms bonding directly to atoms constituting the ring is referred to also as "p-valent aromatic hetero ring group".

"The aromatic heterocyclic compound" includes, for example, compounds in which the hetero ring itself shows aromaticity such as azole, thiophene, furan, pyridine, diazabenzene, triazine, azanaphthalene, diazanaphthalene, carbazole and the like, and, compounds in which an aromatic ring is condensed to a hetero ring even if the hetero ring itself shows no aromaticity such as phenoxazine, phenothiazine, benzopyran and the like.

The number of carbon atoms of the hetero ring group, not including the number of carbon atoms of the substituent, is usually 1 to 60, preferably 2 to 40, and more preferably 3 to 20. The number of hetero atoms of the aromatic hetero ring group, not including the number of hetero atoms of the substituent, is usually 1 to 30, preferably 1 to 10, more preferably 1 to 5, and further preferably 1 to 3.

The hetero ring group includes, for example, groups obtained by removing from a monocyclic heterocyclic compound (including, for example, furan, thiophene, oxadiazole, thiadiazole, pyrrole, diazole, triazole, tetrazole, pyridine, diazabenzene and triazine) or a polycyclic heterocyclic compound (including, for example, dicyclic heterocyclic compounds such as azanaphthalene, diazanaphthalene, benzofuran, benzothiophene, indole, benzodiazole, benzothiadiazole and the like; tricyclic heterocyclic compounds such as dibenzofuran, dibenzothiophene, dibenzoborole, dibenzosilole, dibenzophosphole, dibenzoselenophene, carbazole, azacarbazole, diazacarbazole, phenoxazine, phenothiazine, 9,10-dihydroacridine, 5,10-dihydrophenazine, phenazaborine, phenophosphazine, phenoselenazine, phenazasiline, azaanthracene, diazaanthracene, azaphenanthrene, diazaphenanthrene and the like; tetracyclic heterocyclic compounds such as hexaazatriphenylene, benzocarbazole, benzonaphthofuran, benzonaphthothiophene and the like; pentacyclic heterocyclic compounds such as dibenzocarbazole, indolocarbazole, indenocarbazole and the like; hexacyclic heterocyclic compounds such as carbazolocarbazole, benzoindolocarbazole, benzoindenocarbazole and the like; and, heptacyclic heterocyclic compounds such as dibenzoindolocarbazole and the like) one or more hydrogen atoms bonding directly to atoms constituting the ring. The hetero ring group includes groups obtained by bonding a plurality of these groups. The hetero ring group optionally has a substituent.

"The halogen atom" denotes a fluorine atom, a chlorine atom, a bromine atom or an iodine atom.

"The amino group" optionally has a substituent, and substituted amino groups (namely, secondary amino groups or tertiary amino groups, more preferably, tertiary amino groups) are preferred. The substituent which the amino group has is preferably an alkyl group, a cycloalkyl group, an aryl group or a monovalent hetero ring group. When a plurality of the substituents which the amino group has are present, they may be the same or different and may be combined together to form a ring together with nitrogen atoms to which they are attached.

The substituted amino group includes, for example, a dialkylamino group, a dicycloalkylamino group and a diarylamino group.

The amino group includes, for example, a dimethylamino group, a diethylamino group, a diphenylamino group, a bis(methylphenyl)amino group and a bis(3,5-di-tert-butylphenyl)amino group.

"The alkenyl group" may be any of linear or branched. The number of carbon atoms of the linear alkenyl group, not including the number of carbon atoms of the substituent, is usually 2 to 30, and preferably 3 to 20. The number of carbon atoms of the branched alkenyl group, not including the number of carbon atoms of the substituent, is usually 3 to 30, and preferably 4 to 20.

The number of carbon atoms of "the cycloalkenyl group", not including the number of carbon atoms of the substituent, is usually 3 to 30, and preferably 4 to 20.

The alkenyl group and the cycloalkenyl group optionally have a substituent. The alkenyl group includes, for example, a vinyl group, a 1-propenyl group, a 2-butenyl group, a 3-butenyl group, a 3-pentenyl group, a 4-pentenyl group, a 1-hexenyl group, a 5-hexenyl group and a 7-octenyl group, and groups obtained by substituting a part or all of hydrogen atoms in these groups with a substituent. The cycloalkenyl group includes, for example, a cyclohexenyl group, a cyclohexadienyl group, a cyclooctatrienyl group and a norbornylenyl group, and groups obtained by substituting a part or all of hydrogen atoms in these groups with a substituent.

"The alkynyl group" may be any of linear or branched. The number of carbon atoms of the alkynyl group, not including carbon atoms of the substituent, is usually 2 to 20, and preferably 3 to 20. The number of carbon atoms of the branched alkynyl group, not including carbon atoms of the substituent, is usually 4 to 30, and preferably 4 to 20.

The number of carbon atoms of "the cycloalkynyl group", not including carbon atoms of the substituent, is usually 4 to 30, and preferably 4 to 20.

The alkynyl group and the cycloalkynyl group optionally have a substituent. The alkynyl group includes, for example, an ethynyl group, a 1-propynyl group, a 2-propynyl group, a 2-butynyl group, a 3-butynyl group, a 3-pentynyl group, a 4-pentynyl group, a 1-hexynyl group and a 5-hexynyl group, and groups obtained by substituting a part or all of hydrogen atoms in these groups with a substituent. The cycloalkynyl group includes, for example, a cyclooctynyl group.

"The cross-linkable group" refers to a group capable of generating a new bond by being subjected to a heating treatment, an ultraviolet irradiation treatment, a near-ultraviolet irradiation treatment, a visible light irradiation treatment, an infrared irradiation treatment, a radical reaction and the like. As the cross-linkable group, cross-linkable groups selected from Group A of cross-linkable group (namely, groups represented by any of the formula (XL-1) to the formula (XL-19)) are preferred.

### (Group A of cross-linkable group)

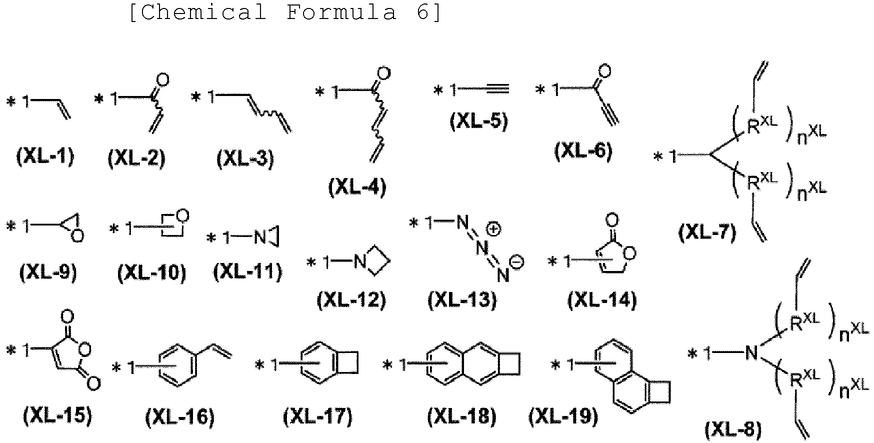

[wherein, R^{XL} represents a methylene group, an oxygen atom or a sulfur atom, and n^{XL} represents an integer of 0 to 5. When a plurality of R^{XL} are present, they may be the same or different. A plurality of n^{XL} may be the same or different. *1 represents a binding position. These cross-linkable groups optionally have a substituent. When a plurality of the substituents are present, they may be the same or different and may be combined together to form a ring together with carbon atoms to which they are attached.]

"The substituent" includes, for example, a halogen atom, a cyano group, an alkyl group, a cycloalkyl group, an aryl group, a monovalent hetero ring group, an alkoxy group, a cycloalkoxy group, an aryloxy group, an amino group, a substituted amino group, an alkenyl group, a cycloalkenyl group, an alkynyl group and a cycloalkynyl group. The substituent may be a cross-linkable group. When a plurality of the substituents are present, they may be combined together to form a ring together with atoms to which they are attached, but it is preferable that they do not form a ring.

In the present specification, calculation of the value of the absolute value of a difference between the energy level of the lowest triplet excited state and the energy level of the lowest singlet excited state (hereinafter, referred to also as "ΔE_{ST}") is carried out by the following method. First, the ground state of a compound is structurally optimized by density-functional approach of B3LYP level. In this procedure, 6-31G* is used as the basis function. Using the resultant structurally optimized structure, ΔE_{ST} of the compound is calculated by B3LYP level time-dependent density-functional approach. In the case of containing an atom to which 6-31G* cannot be applied, LANL2DZ is used for the atom. Calculation is performed using Gaussian09 as the quantum chemistry calculation program.

### <Composition for light emitting device>

The composition for light emitting device of the present embodiment contains two or more compounds (A) and a compound (B).

The composition for light emitting device of the present embodiment may contain only two compounds (A), or may contain three or more compounds (A). Meanwhile, the composition for light emitting device of the present embodiment may contain only one compound (B), or may contain two or more compounds (B).

In the composition for light emitting device of the present embodiment, the compound (A) is preferably a host material and the compound (B) is preferably a guest material. In the present embodiment, the host material is a material interacting physically, chemically or electrically with a guest material. By this interaction, it becomes possible to improve or adjust, for example, the light emitting property, the charge transporting property or the charge injection property of the composition for light emitting device of the present embodiment.

In the composition for light emitting device of the present embodiment, if a light emitting material is explained as an example, the host material and the guest material interact electrically to transfer electric energy efficiently from the host material to the guest material, accordingly, the guest material can be allowed to emit light more efficiently, and the light emitting device of the present embodiment is more excellent in external quantum efficiency.

When the compound (B) is used as a light emitting material, the maximum peak wavelength of the emission spectrum at 25°C of the compound (B) is preferably in the visible light region. In this case, the maximum peak wavelength of the emission spectrum at 25°C of the compound (B) is preferably 380 nm or more, more preferably 400 nm or more, further preferably 420 nm or more, and particularly preferably 440 nm or more. The maximum peak wavelength of the emission spectrum at 25°C of the compound (B) is preferably 750 nm or less, more preferably 620 nm or less, further preferably 570 nm or less, particularly preferably 495 nm or less, and especially preferably 480 nm or less. Further, when the compound (B) is used as a light emitting material, the half-value width of the maximum peak of the emission spectrum at 25°C of the compound (B) is preferably 50 nm or less, more preferably 40 nm or less, further preferably 30 nm or less, and particularly preferably 25 nm or less.

The maximum peak wavelength of the emission spectrum at room temperature of the compound can be evaluated by dissolving the compound in an organic solvent such as xylene, toluene, chloroform, tetrahydrofuran and the like to prepare a dilute solution (1×10⁻⁶% by mass to 1×10⁻³% by mass), and measuring the PL spectrum of the dilute solution at room temperature. As the organic solvent for dissolving the compound, xylene is preferable.

In the composition for light emitting device of the present embodiment, the content of the compound (B) is usually 0.001 to 99 parts by mass, when the sum of the compound (A) and the compound (B) is taken as 100 parts by mass, and it is preferably 0.005 to 70 parts by mass, more preferably 0.01 to 50 parts by mass, further preferably 0.05 to 30 parts by mass, particularly preferably 0.1 to 10 parts by mass, and especially preferably 0.5 to 5 parts by mass, since the light emitting device of the present embodiment is more excellent in external quantum efficiency.

In the composition for light emitting device of the present embodiment, the content of at least one of compounds (A) is usually 0.01 to 99 parts by mass, when the sum of the compounds (A) is taken as 100 parts by mass, and it is preferably 0.05 to 90 parts by mass, more preferably 0.1 to 70 parts by mass, further preferably 0.5 to 50 parts by mass, particularly preferably 1 to 30 parts by mass, and especially preferably 3 to 10 parts by mass, since the light emitting device of the present embodiment is more excellent in external quantum efficiency.

### (Compound (A))

The compound (A) is a compound represented by the formula (FH), or a polymer compound (hereinafter, referred to also as "polymer compound (A)") containing a constitutional unit (hereinafter, referred to also as "constitutional unit (A)") having a group obtained by removing from a compound represented by the formula (FH) one or more hydrogen atoms. The compound (A) is a compound different from the compound (B), and may be, for example, a compound having no condensed hetero ring skeleton (b).

The composition for light emitting device of the present embodiment contains at least two compounds (A). The composition for light emitting device of the present embodiment may contain two or more compounds represented by the formula (FH), may contain one or more compounds represented by the formula (FH) and one or more polymer compounds (A), or may contain two or more polymer compounds (A). The composition for light emitting device of the present embodiment preferably contains at least one polymer compound (A) as the compound (A), since the light emitting device is more excellent in external quantum efficiency. That is, the composition for light emitting device of the present embodiment preferably contains one or more compounds represented by the formula (FH) and one or more polymer compounds (A), or contains two or more polymer compounds (A).

### [Compound represented by the formula (FH)]

n^{1H} is usually an integer of 10 or less, and it is preferably an integer of 7 or less, more preferably an integer of 5 or less, and further preferably an integer of 3 or less, since synthesis of a compound represented by the formula (FH) is easy. Meanwhile, n^{1H} is preferably an integer of 1 or more, since the light emitting device of the present embodiment is more excellent in external quantum efficiency.

Ar^{1H} is a group obtained by removing from a heterocyclic compound containing a nitrogen atom in the ring n^{1H} or more hydrogen atoms bonding directly to atoms constituting the ring (hereinafter, referred to also as "nitrogen-containing hetero ring group"), and this group optionally has a substituent.

The heterocyclic compound in Ar^{1H} is a heterocyclic compound containing no condensed hetero ring skeleton (b). That is, the heterocyclic compound in Ar^{1H} is a heterocyclic compound containing a nitrogen atom in the ring and containing no boron atom in the ring. The heterocyclic compound in Ar^{1H} includes, for example, heterocyclic compounds containing a nitrogen atom in the ring and containing no boron atom in the ring among heterocyclic compounds explained in the section of the hetero ring group described above.

The number of carbon atoms of the nitrogen-containing hetero ring group represented by Ar^{1H}, not including the number of carbon atoms of the substituent, is preferably 1 to 60, more preferably 1 to 40, and further preferably 2 to 20.

The number of nitrogen atoms of the nitrogen-containing hetero ring group represented by Ar^{1H}, not including the number of nitrogen atoms of the substituent, is preferably 1 to 10, more preferably 1 to 5, and further preferably 1 to 3. The number of hetero atoms of the nitrogen-containing hetero ring group represented by Ar^{1H}, not including the number of hetero atoms of the substituent, is preferably 1 to 10, more preferably 1 to 5, and further preferably 1 to 3.

The heterocyclic compound in Ar^{1H} is preferably a monocyclic or di to heptacyclic heterocyclic compound containing a nitrogen atom in the ring and containing no boron atom in the ring, more preferably a monocyclic or di to pentacyclic heterocyclic compound containing a nitrogen atom in the ring and containing no boron atom in the ring, further preferably a monocyclic, dicyclic or tricyclic heterocyclic compound containing a nitrogen atom in the ring and containing no boron atom in the ring, particularly preferably oxadiazole, thiadiazole, pyrrole, diazole, triazole, pyridine, diazabenzene, triazine, azanaphthalene, diazanaphthalene, indole, benzodiazole, carbazole, azacarbazole, diazacarbazole, phenoxazine, phenothiazine, 9,10-dihydroacridine, 5,10-dihydrophenazine, azaanthracene, diazaanthracene, azaphenanthrene or diazaphenanthrene, especially preferably pyrrole, diazole, triazole, pyridine, diazabenzene, triazine, carbazole, azacarbazole, diazacarbazole, phenoxazine, phenothiazine, 9,10-dihydroacridine or 5,10-dihydrophenazine, and especially more preferably pyridine, diazabenzene, triazine, carbazole, phenoxazine or phenothiazine, since the light emitting device of the present embodiment is more excellent in external quantum efficiency.

The substituent which Ar^{1H} optionally has includes, for example, substituents other than the aryl group, the monovalent hetero ring group and the substituted amino group, and is preferably a halogen atom, a cyano group, an alkyl group, a cycloalkyl group, an alkoxy group, a cycloalkoxy group, an alkenyl group or a cycloalkenyl group, more preferably a cyano group, an alkyl group, a cycloalkyl group, an alkoxy group or a cycloalkoxy group, further preferably an alkyl group, a cycloalkyl group, an alkoxy group or a cycloalkoxy group, and particularly preferably an alkyl group or a cycloalkyl group, and these groups optionally further have a substituent.

The examples and preferable ranges of the substituent which the substituent which Ar^{1H} optionally has optionally further has are the same as the examples and preferable ranges of the substituent which the substituent which R^{1A} optionally has optionally further has described later.

R^{1H} is preferably an aryl group or a monovalent hetero ring group, and more preferably an aryl group, since the light emitting device of the present embodiment is more excellent in external quantum efficiency, and these groups optionally have a substituent.

The aryl group represented by R^{1H} is preferably a group obtained by removing from a monocyclic or dicyclic to pentacyclic aromatic hydrocarbon one hydrogen atom bonding directly to an atom constituting the ring, more preferably a group obtained by removing from a monocyclic, dicyclic or tricyclic aromatic hydrocarbon one hydrogen atom bonding directly to an atom constituting the ring, further preferably a group obtained by removing from benzene, naphthalene, dihydrophenanthrene or fluorene one hydrogen atom bonding directly to an atom constituting the ring, and particularly preferably a phenyl group, and these groups optionally have a substituent.

The monovalent hetero ring group represented by R^{1H} is a group obtained by removing from a heterocyclic compound containing no condensed hetero ring skeleton (b) one hydrogen atom bonding directly to an atom constituting the ring, and this group optionally has a substituent. In the monovalent hetero ring group represented by R^{1H}, the heterocyclic compound containing no condensed hetero ring skeleton (b) includes heterocyclic compounds not containing a boron atom and a nitrogen atom in the ring among heterocyclic compounds explained in the section of the hetero ring group described above. The monovalent hetero ring group represented by R^{1H} is preferably a group obtained by removing from a monocyclic or dicyclic to pentacyclic heterocyclic compound containing no condensed hetero ring skeleton (b) one hydrogen atom bonding directly to an atom constituting the ring, more preferably a group obtained by removing from a monocyclic, dicyclic or a tricyclic heterocyclic compound containing no condensed hetero ring skeleton (b) one hydrogen atom bonding directly to an atom constituting the ring, further preferably a group obtained by removing from pyrrole, diazole, triazole, pyridine, diazabenzene, triazine, azanaphthalene, diazanaphthalene, dibenzofuran, dibenzothiophene, carbazole, azacarbazole, diazacarbazole, phenoxazine, phenothiazine, 9,10-dihydroacridine, 5,10-dihydrophenazine, azaanthracene, diazaanthracene, azaphenanthrene or diazaphenanthrene one hydrogen atom bonding directly to an atom constituting the ring, and particularly preferably a group obtained by removing from pyridine, diazabenzene, triazine, dibenzofuran, dibenzothiophene or carbazole one hydrogen atom bonding directly to an atom constituting the ring, and these groups optionally have a substituent.

In the substituted amino group represented by R^{1H}, the substituent which the amino group has is preferably an aryl group or a monovalent hetero ring group, and more preferably an aryl group, and these groups optionally further have a substituent. The examples and preferable ranges of the aryl group as the substituent which the amino group has are the same as the examples and preferable ranges of the aryl group represented by R^{1H}. The examples and preferable ranges of the monovalent hetero ring group as the substituent which the amino group has are the same as the examples and preferable ranges of the monovalent hetero ring group represented by R^{1H}.

The substituent which R^{1H} optionally has is preferably a halogen atom, a cyano group, an alkyl group, a cycloalkyl group, an alkoxy group, a cycloalkoxy group, an aryl group, a monovalent hetero ring group or a substituted amino group, more preferably an alkyl group, a cycloalkyl group, an aryl group, a monovalent hetero ring group or a substituted amino group, and further preferably an alkyl group, a cycloalkyl group or an aryl group, and these groups optionally further have a substituent.

The examples and preferable ranges of the aryl group, the monovalent hetero ring group and the substituted amino group as the substituent which R^{1H} optionally has are the same as the examples and preferable ranges of the aryl group, the monovalent hetero ring group and the substituted amino group represented by R^{1H}.

The substituent which the substituent which R^{1H} optionally has optionally further has is preferably a halogen atom, a cyano group, an alkyl group, a cycloalkyl group, an alkoxy group, a cycloalkoxy group, an aryl group, a monovalent hetero ring group or a substituted amino group, more preferably an alkyl group, a cycloalkyl group, an aryl group, a monovalent hetero ring group or a substituted amino group, and further preferably an alkyl group or a cycloalkyl group, and these groups optionally further have a substituent, but it is preferable that they do not further have a substituent.

The examples and preferable ranges of the aryl group, the monovalent hetero ring group and the substituted amino group as the substituent which the substituent which R^{1H} optionally has optionally further has are the same as the examples and preferable ranges of the aryl group, the monovalent hetero ring group and the substituted amino group represented by R^{1H}.

As the compound (A), compounds represented by the following formulae and compounds H2 to H8 described later are exemplified. In the formulae, Z¹ represents an oxygen atom or a sulfur atom. In the formulae, Z² represents a group represented by -CH= or a group represented by -N=.

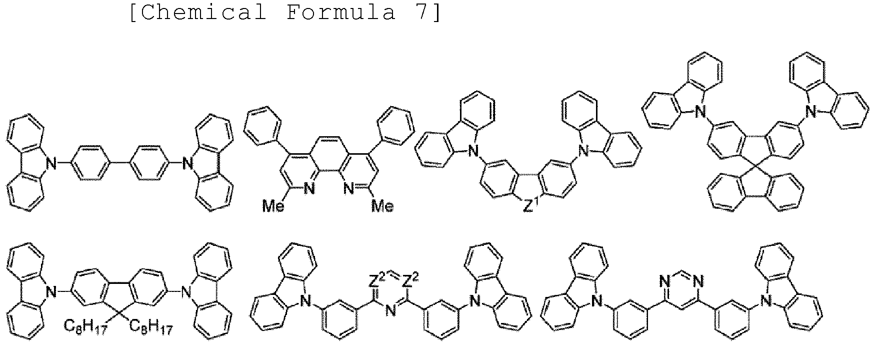

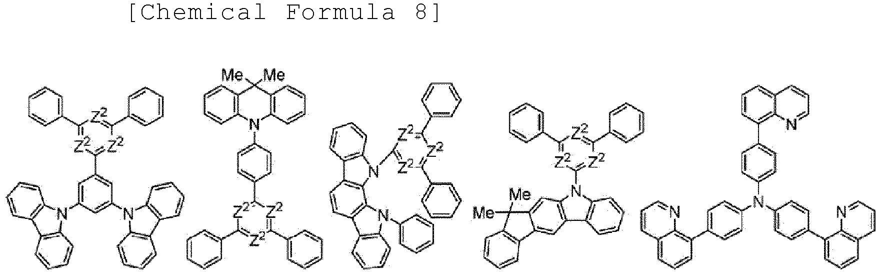

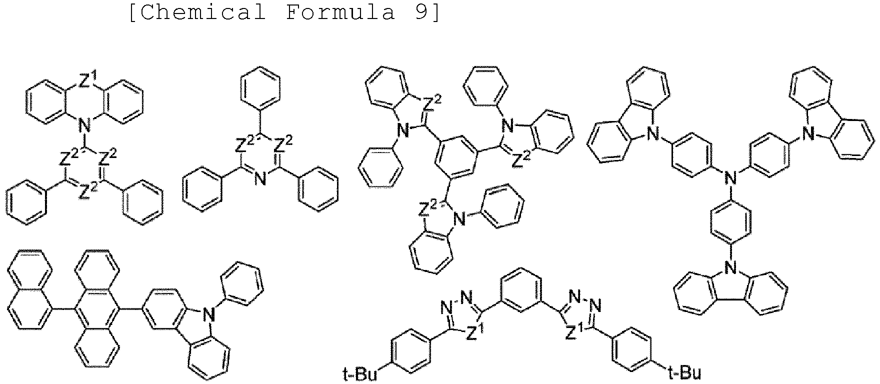

### [Polymer compound (A)]

The polymer compound (A) has a polystyrene-equivalent weight-average molecular weight of preferably 1×10⁴ to 1×10⁷, more preferably 2×10⁴ to 5×10⁶, and further preferably 1×10⁵ to 2×10⁵.

The polymer compound (A) may be any of a block copolymer, a random copolymer, an alternating copolymer and a graft copolymer, and may also be another form, and it is preferably a copolymer obtained by copolymerizing a plurality of kinds of raw material monomers.

The constitutional unit (A) is preferably a constitutional unit having a group obtained by removing from a compound represented by the formula (FH) one or more and five or less hydrogen atoms, more preferably a constitutional unit having a group obtained by removing from a compound represented by the formula (FH) one or more and three or less hydrogen atoms, and further preferably a constitutional unit having a group obtained by removing from a compound represented by the formula (FH) one or two hydrogen atoms, since synthesis of the polymer compound (A) is easy.

The constitutional unit (A) is preferably a constitutional unit represented by the formula (FH-1), the formula (FH-2) or the formula (FH-3), and more preferably a constitutional unit represented by the formula (FH-1) or the formula (FH-2), since synthesis of the polymer compound (A) is easy and the light emitting device of the present embodiment is more excellent in external quantum efficiency.

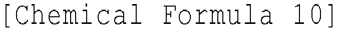

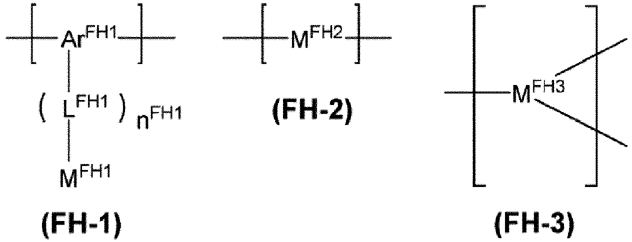

In the formulae,
M^{FH1} represents a group obtained by removing from a compound represented by the formula (FH) one hydrogen atom.
M^{FH2} represents a group obtained by removing from a compound represented by the formula (FH) two hydrogen atoms.
M^{FH3} represents a group obtained by removing from a compound represented by the formula (FH) three hydrogen atoms.
L^{FH1} represents an alkylene group, a cycloalkylene group, an arylene group, a divalent hetero ring group, a group represented by -N(R^{FH1})-, an oxygen atom or a sulfur atom, and these groups optionally have a substituent. When a plurality of the substituents are present, they may be the same or different and may be combined together to form a ring together with atoms to which they are attached. R^{FH1} represents a hydrogen atom, an alkyl group, a cycloalkyl group, an aryl group or a monovalent hetero ring group, and these groups optionally have a substituent. When a plurality of the substituents are present, they may be the same or different and may be combined together to form a ring together with atoms to which they are attached. When a plurality of L^{FH1} are present, they may be the same or different.
n^{FH1} represents an integer of 0 or more and 10 or less.
Ar^{FH1} represents a hydrocarbon group or a hetero ring group, and these groups optionally have a substituent. When a plurality of the substituents are present, they may be the same or different and may be combined together to form a ring together with atoms to which they are attached.
L^{FH1} is preferably an alkylene group, a cycloalkylene group, an arylene group or a divalent hetero ring group, and more preferably an alkylene group or an arylene group, and these groups optionally have a substituent.

The examples and preferable ranges of the arylene group and the divalent hetero ring group represented by L^{FH1} are the same as the examples and preferable ranges of the arylene group and the divalent hetero ring group represented by Ar^{Y1}, respectively.

The examples and preferable ranges of the R^{FH1} are the same as the examples and preferable ranges of the R^{X1} described later.

n^{FH1} is preferably an integer of 0 to 5, preferably an integer of 0 to 3, more preferably 0 or 1, and further preferably 0.

The hydrocarbon group represented by Ar^{FH1} is a group obtained by removing three hydrogen atoms from an aliphatic hydrocarbon or an aromatic hydrocarbon, and this group optionally has a substituent.

When the hydrocarbon group represented by Ar^{FH1} is a group obtained by removing three hydrogen atoms from an aliphatic hydrocarbon, the hydrocarbon group includes groups obtained by removing one hydrogen atom from alkylene groups explained in the section of the alkylene group described above, and groups obtained by removing one hydrogen atom from cycloalkylene groups explained in the section of the cycloalkylene group described above.

When the hydrocarbon group represented by Ar^{FH1} is a group obtained by removing three hydrogen atoms from an aromatic hydrocarbon, the hydrocarbon group includes groups obtained by removing one hydrogen atom from groups explained as the arylene group represented by Ar^{Y1} described later.

The hetero ring group represented by Ar^{FH1} includes groups obtained by removing one hydrogen atom from groups explained as the divalent hetero ring group represented by Ar^{Y1} described later.

The examples and preferable ranges of the substituent which L^{FH1} and Ar^{FH1} optionally have are the same as the examples and preferable ranges of the substituent which the group represented by Ar^{Y1} optionally has described later.

The constitutional unit (A) includes, for example, constitutional units represented by the following formulae, and constitutional units derived from compounds H1 to H8 and compound M6 described later. In the formulae, Z¹ represents an oxygen atom or a sulfur atom. In the formulae, Z² represents a group represented by -CH= or a group represented by -N=.

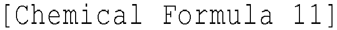

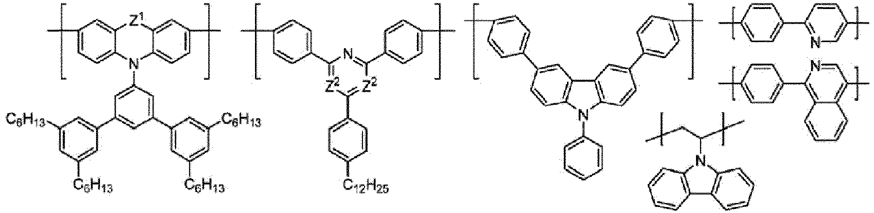

The content of the constitutional unit (A) is preferably 0.1 to 100% by mol, more preferably 1 to 100% by mol, further preferably 10 to 100% by mol, and particularly preferably 20 to 100% by mol, with respect to the total amount of constitutional units contained in the polymer compound (A), since the light emitting device of the present embodiment is more excellent in external quantum efficiency. The constitutional unit (A) may be contained singly or in combination of two or more in the polymer compound (A).

The polymer compound (A) preferably further contains a constitutional unit represented by the formula (Y) described later, since the light emitting device of the present embodiment is more excellent in external quantum efficiency.

The polymer compound (A) may further contain a constitutional unit represented by the formula (X) described later.

The polymer compound (A) may further contain the constitutional unit represented by the formula (X) and the constitutional unit represented by the formula (Y).

When the polymer compound (A) contains a constitutional unit represented by the formula (X), the content of the constitutional unit represented by the formula (X) is preferably 0.1 to 99% by mol, more preferably 1 to 50% by mol, and further preferably 3 to 30% by mol, with respect to the total amount of constitutional units contained in the polymer compound (A), since hole transportability is excellent.

The constitutional unit represented by the formula (X) may be contained singly or in combination of two or more in the polymer compound (A).

If the polymer compound (A) contains a constitutional unit represented by the formula (Y) and Ar^{Y1} is an arylene group, then, the content of the constitutional unit represented by the formula (Y) is preferably 1 to 99% by mol, more preferably 10 to 95% by mol, further preferably 30 to 90% by mol, and particularly preferably 50 to 80% by mol, with respect to the total amount of the polymer compound (A), since the light emitting device of the present embodiment is more excellent in external quantum efficiency.

If the polymer compound (A) contains a constitutional unit represented by the formula (Y) and Ar^{Y1} is a divalent hetero ring group or a divalent group in which at least one arylene group and at least one divalent hetero ring group are bonded directly, then, the content of the constitutional unit represented by the formula (Y) is preferably 0.1 to 99% by mol, more preferably 1 to 50% by mol, and further preferably 3 to 30% by mol, with respect to the total amount of constitutional units contained in the polymer compound (A), since the polymer compound (A) is excellent in charge transportability.

The constitutional unit represented by the formula (Y) may be contained singly or in combination of two or more in the polymer compound (A).

### <Constitutional unit represented by the formula (Y)>

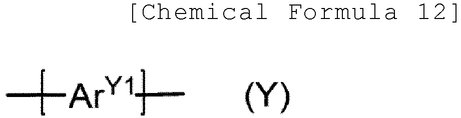

In the formula, Ar^{Y1} represents an arylene group, a divalent hetero ring group, or a divalent group in which at least one arylene group and at least one divalent hetero ring group are bonded directly, and these groups optionally have a substituent. When a plurality of the substituents are present, they may be the same or different and may be combined together to form a ring together with atoms to which they are attached.

The arylene group represented by Ar^{Y1} is preferably a group obtained by removing from a monocyclic or dicyclic to hexacyclic aromatic hydrocarbon two hydrogen atoms bonding directly to atoms constituting the ring, more preferably a group obtained by removing from a monocyclic, dicyclic or tricyclic aromatic hydrocarbon two hydrogen atoms bonding directly to atoms constituting the ring, further preferably a group obtained by removing from benzene, naphthalene, anthracene, phenanthrene, dihydrophenanthrene or fluorene two hydrogen atoms bonding directly to atoms constituting the ring, and particularly preferably a group obtained by removing from benzene, phenanthrene, dihydrophenanthrene or fluorene two hydrogen atoms bonding directly to atoms constituting the ring, since the light emitting device of the present embodiment is more excellent in external quantum efficiency, and these groups optionally have a substituent.

The divalent hetero ring group represented by Ar^{Y1} is preferably a group obtained by removing from a heterocyclic compound containing no nitrogen atom in the ring two hydrogen atoms bonding directly to atoms (preferably, carbon atoms) constituting the ring, and this group optionally has a substituent.

The heterocyclic compound containing no nitrogen atom in the ring includes, for example, heterocyclic compounds containing no nitrogen atom in the ring among heterocyclic compounds explained in the section of the hetero ring group described above. The heterocyclic compound containing no nitrogen atom in the ring is preferably a monocyclic or dicyclic to hexacyclic heterocyclic compound containing no nitrogen atom in the ring, more preferably a monocyclic, dicyclic or tricyclic heterocyclic compound containing no nitrogen atoms in the ring, and further preferably furan, thiophene, benzofuran, benzothiophene, dibenzofuran or dibenzothiophene, since the light emitting device of the present embodiment is more excellent in external quantum efficiency.

The preferable ranges of the arylene group and the divalent hetero ring group as the divalent group in which at least one arylene group and at least one divalent hetero ring group are bonded directly represented by Ar^{Y1} are the same as the preferable ranges of the arylene group and the divalent hetero ring group represented by Ar^{Y1}, respectively.

Ar^{Y1} is preferably an arylene group optionally having a substituent, since the light emitting device of the present embodiment is more excellent in external quantum efficiency.

The substituent which the group represented by Ar^{Y1} optionally has is preferably an alkyl group, a cycloalkyl group, an alkoxy group, a cycloalkoxy group, an aryl group, an aryloxy group, a monovalent hetero ring group, a substituted amino group or a fluorine atom, more preferably an alkyl group, a cycloalkyl group, an alkoxy group, a cycloalkoxy group, an aryl group, a monovalent hetero ring group or a substituted amino group, further preferably an alkyl group, a cycloalkyl group, an aryl group, a monovalent hetero ring group or a substituted amino group, and particularly preferably an alkyl group, a cycloalkyl group or an aryl group, and these groups optionally further have a substituent.

The aryl group as the substituent which the group represented by Ar^{Y1} optionally has is preferably a group obtained by removing from a monocyclic or dicyclic to hexacyclic aromatic hydrocarbon one hydrogen atom bonding directly to an atom constituting the ring, more preferably a group obtained by removing from a monocyclic, dicyclic or tricyclic aromatic hydrocarbon one hydrogen atom bonding directly to an atom constituting the ring, further preferably a group obtained by removing from benzene, naphthalene, anthracene, phenanthrene, dihydrophenanthrene or fluorene one hydrogen atom bonding directly to an atom constituting the ring, and particularly preferably a group obtained by removing from benzene, phenanthrene, dihydrophenanthrene or fluorene one hydrogen atom bonding directly to an atom constituting the ring, since the light emitting device of the present embodiment is more excellent in external quantum efficiency, and these groups optionally further have a substituent.

The monovalent hetero ring group as the substituent which the group represented by Ar^{Y1} optionally has is preferably a group obtained by removing from a heterocyclic compound containing no nitrogen atom in the ring one hydrogen atom bonding directly to an atom (preferably, a carbon atom) constituting the ring, and this group optionally has a substituent.

The examples and preferable ranges of the heterocyclic compound containing no nitrogen atom in the ring for the monovalent hetero ring group as the substituent which the group represented by Ar^{Y1} optionally has are the same as the examples and preferable ranges of the heterocyclic compound containing no nitrogen atom in the ring explained in the section of the divalent hetero ring group represented by Ar^{Y1}.

In the substituted amino group as the substituent which the group represented by Ar^{Y1} optionally has, the substituent which the amino group has is preferably an aryl group or a monovalent hetero ring group, and more preferably an aryl group, and these groups optionally further have a substituent. The examples and preferable ranges of the aryl group and the monovalent hetero ring group as the substituent which the amino group has are the same as the examples and preferable ranges of the aryl group and the monovalent hetero ring group as the substituent which the group represented by Ar^{Y1} optionally has, respectively.

The substituent which the substituent which the group represented by Ar^{Y1} optionally has optionally further has is preferably an alkyl group, a cycloalkyl group, an alkoxy group, a cycloalkoxy group, an aryl group, an aryloxy group, a monovalent hetero ring group, a substituted amino group or a fluorine atom, more preferably an alkyl group, a cycloalkyl group, an aryl group, a monovalent hetero ring group or a substituted amino group, further preferably an alkyl group or a cycloalkyl group, and these groups optionally further have a substituent, but it is preferable that they do not further have a substituent.

The examples and preferable ranges of the aryl group, the monovalent hetero ring group and the substituted amino group as the substituent which the substituent which the group represented by Ar^{Y1} optionally has optionally further has are the same as the examples and preferable ranges of the aryl group, the monovalent hetero ring group and the substituted amino group as the substituent which the group represented by Ar^{Y1} optionally has, respectively.

The constitutional unit represented by the formula (Y) is preferably a constitutional unit represented by the formula (Y-1) or the formula (Y-2), since the light emitting device of the present embodiment is more excellent in external quantum efficiency.

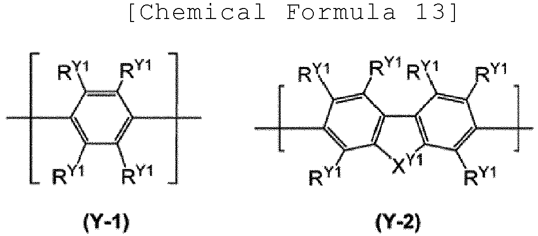

In the formulae,
R^{Y1} represents a hydrogen atom, an alkyl group, a cycloalkyl group, an alkoxy group, a cycloalkoxy group, an aryl group, an aryloxy group, a monovalent hetero ring group, a substituted amino group or a fluorine atom, and these groups optionally have a substituent. When a plurality of the substituents are present, they may be the same or different and may be combined together to form a ring together with atoms to which they are attached. A plurality of R^{Y1} may be the same or different and may be combined together to form a ring together with carbon atoms to which they are attached.
X^{Y1} represents a group represented by -C(RY²)₂-, - C(RY²)=C(RY²)- or -C(RY²)₂-C(RY²)₂-. RY² represent a hydrogen atom, an alkyl group, a cycloalkyl group, an alkoxy group, a cycloalkoxy group, an aryl group, an aryloxy group, a monovalent hetero ring group, a substituted amino group or a fluorine atom, and these groups optionally have a substituent. When a plurality of the substituents are present, they may be the same or different and may be combined together to form a ring together with atoms to which they are attached. A plurality of RY² may be the same or different and may be combined together to form a ring together with carbon atoms to which they are attached.
R^{Y1} is preferably a hydrogen atom, an alkyl group, a cycloalkyl group, an aryl group, a monovalent hetero ring group or a substituted amino group, more preferably a hydrogen atom, an alkyl group, a cycloalkyl group or an aryl group, and further preferably a hydrogen atom or an alkyl group, and these groups optionally have a substituent.
R^{Y2} is preferably an alkyl group, a cycloalkyl group, an alkoxy group, a cycloalkoxy group, an aryl group, a monovalent hetero ring group or a substituted amino group, more preferably an alkyl group, a cycloalkyl group, an aryl group or a monovalent hetero ring group, further preferably an alkyl group, a cycloalkyl group or an aryl group, and these groups optionally have a substituent.

The examples and preferable ranges of the aryl group, the monovalent hetero ring group and the substituted amino group represented by R^{Y1} and RY² are the same as the examples and preferable ranges of the aryl group, the monovalent hetero ring group and the substituted amino group as the substituent which the group represented by Ar^{Y1} optionally has, respectively.

The examples and preferable ranges of the substituent which R^{Y1} and R^{Y2} optionally have are the same as the examples and preferable ranges of the substituent which the group represented by Ar^{Y1} optionally has.

X^{Y1} is preferably a group represented by -C(R^{Y2})₂- or -C(R^{Y2})₂-C(R^{Y2})₂-, and more preferably a group represented by -C(R^{Y2})₂-, since the light emitting device of the present embodiment is more excellent in external quantum efficiency.

The constitutional unit represented by the formula (Y) includes, for example, constitutional units represented by the following formulae, and constitutional units derived from compounds M1, M2, M4 and M5 described later. In the following formulae, Z¹ represents an oxygen atom or a sulfur atom. Z² represents a group represented by -CH= or a group represented by -N=.

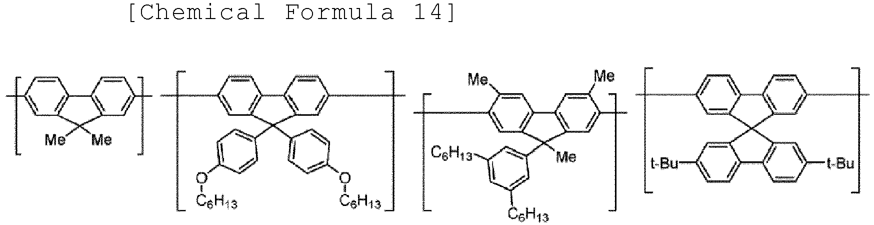

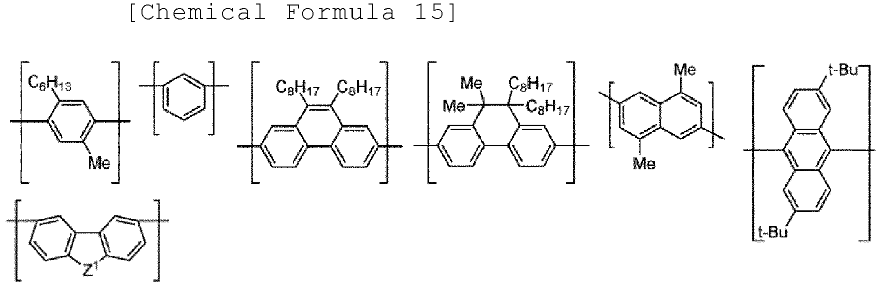

### <Constitutional unit represented by the formula (X)>

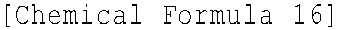

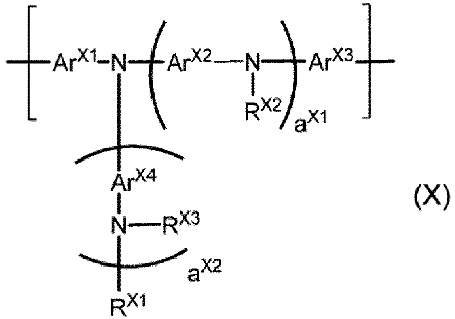

In the formula,
a^{X1} and a^{X2} each independently represent an integer of 0 or more.
Ar^{X1} and Ar^{X3} each independently represent an arylene group or a divalent hetero ring group, and these groups optionally have a substituent. When a plurality of the substituents are present, they may be the same or different and may be combined together to form a ring together with atoms to which they are attached.
Ar^{X2} and Ar^{X4} each independently represent an arylene group, a divalent hetero ring group, or a divalent group in which at least one arylene group and at least one divalent hetero ring group are bonded directly, and these groups optionally have a substituent. When a plurality of the substituents are present, they may be the same or different and may be combined together to form a ring together with atoms to which they are attached. When a plurality of Ar^{X2} and Ar^{X4} are present, they may be the same or different at each occurrence.
R^{X1}, R^{X2} and R^{X3} each independently represent a hydrogen atom, an alkyl group, a cycloalkyl group, an aryl group or a monovalent hetero ring group, and these groups optionally have a substituent. When a plurality of the substituents are present, they may be the same or different and may be combined together to form a ring together with atoms to which they are attached. When a plurality of R^{X2} and R^{X3} are present, they may be the same or different at each occurrence.
a^{X1} and a^{X2} are each usually an integer of 0 to 5, and each preferably an integer of 0 to 3, more preferably an integer of 0 to 2, and more preferably 0 or 1, since the light emitting device of the present embodiment is more excellent in external quantum efficiency.

R^{X1}, R^{X2} and R^{X3} represent preferably an alkyl group, a cycloalkyl group, an aryl group or a monovalent hetero ring group, more preferably an aryl group or a monovalent hetero ring group, and further preferably an aryl group, and these groups optionally have a substituent.

The examples and preferable ranges of the aryl group and the monovalent hetero ring group represented by R^{X1}, R^{X2} and R^{X3} are the same as the examples and preferable ranges of the aryl group and the monovalent hetero ring group as the substituent which the group represented by Ar^{Y1} optionally has, respectively.

The examples and preferable ranges of the arylene group and the divalent hetero ring group represented by Ar^{X1}, Ar^{X2}, Ar^{X3} and Ar^{X4} are the same as the examples and preferable ranges of the arylene group and the divalent hetero ring group the represented by Ar^{Y1}, respectively.

The examples and preferable ranges of the arylene group and the divalent hetero ring group as the divalent group in which at least one arylene group and at least one divalent hetero ring group are bonded directly represented by Ar^{X2} and Ar^{X4} are the same as the examples and preferable ranges of the arylene group and the divalent hetero ring group the represented by Ar^{Y1}, respectively.

The divalent group in which at least one arylene group and at least one divalent hetero ring group are bonded directly represented by Ar^{X2} and Ar^{X4} includes the same groups as the divalent group in which at least one arylene group and at least one divalent hetero ring group are bonded directly represented by Ar^{Y1}.

Ar^{X1}, Ar^{X2}, Ar^{X3} and Ar^{X4} are each preferably an arylene group optionally having a substituent.

The examples and preferable ranges of the substituent which the group represented by Ar^{X1} to Ar^{X4} and R^{X1} to R^{X3} optionally has are the same as the examples and preferable ranges of the substituent which the group represented by Ar^{Y1} optionally has.

The constitutional unit represented by the formula (X) includes, for example, constitutional units represented by the following formulae and constitutional units derived from a compound M3 described later.

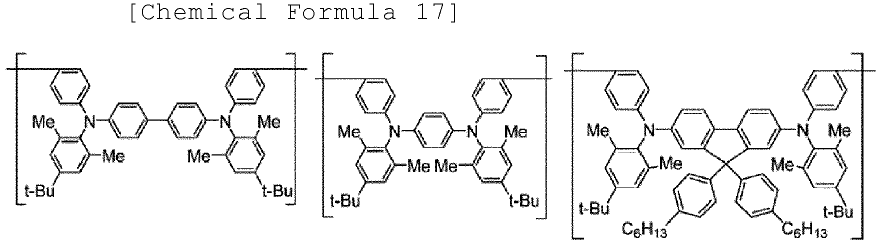

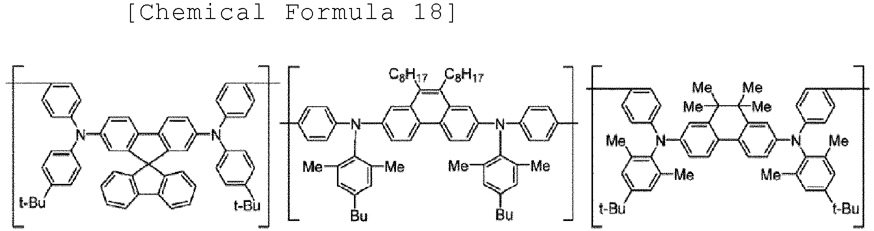

The polymer compound (A) includes, for example, polymer compounds P-1 to P-4. "Other" means a constitutional unit other than the constitutional unit (A), the constitutional unit represented by the formula (X) and the constitutional unit represented by the formula (Y).

**[Table 1]**

| Polymer compound | Constitutional unit and its molar ratio | | | |
|---|---|---|---|---|
| | Constitutional unit A | Formula (X) | Formula (Y) | other |
| | p' | q' | r' | s' |
| P-1 | 0.1~100 | 0 | 0 | 0~30 |
| P-2 | 0.1~99.9 | 0.1~99.9 | 0 | 0~30 |
| P-3 | 0.1~99.9 | 0 | 0.1~99.9 | 0~30 |
| P-4 | 0.1~99.8 | 0.1~99.8 | 0.1~99.8 | 0~30 |

In Table 1, p', q', r' and s' represent the molar ratio (% by mol) of each constitutional unit. p'+q'+r'+s' = 100, and 70 ≤ p'+q'+r' ≤ 100.

The polymer compound (A) may be any of a block copolymer, a random copolymer, an alternating copolymer and a graft copolymer, and may also be another form, and it is preferably a copolymer obtained by copolymerizing a plurality of kinds of raw material monomers.

The polymer compound (A) has a polystyrene-equivalent weight-average molecular weight of preferably 1×10⁴ to 1×10⁷, more preferably 2×10⁴ to 5×10⁶, and further preferably 1×10⁵ to 2×10⁵.

### [Production method of polymer compound (A)]

The polymer compound (A) can be produced using known polymerization methods described in Chemical Reviews (Chem. Rev.), vol. 109, pp. 897-1091 (2009) and the like, and methods of polymerizing by a coupling reaction using a transition metal catalyst such as in the Suzuki reaction, the Yamamoto reaction, the Buchwald reaction, the Stille reaction, the Negishi reaction, the Kumada reaction and the like are exemplified.

In the above-described polymerization method, the method of charging monomers includes a method of charging the entire amount of monomers into the reaction system at once, a method in which a part of the monomers is charged and reacted, then, the remaining monomers are charged in a batch, continuously or dividedly, a method of charging monomers continuously or dividedly, and the like.

The transition metal catalyst includes palladium catalysts, nickel catalysts and the like.

The post treatment of the polymerization reaction is performed using known methods, for example, a method of removing water-soluble impurities by liquid separation, a method in which the reaction liquid after the polymerization reaction is added to a lower alcohol such as methanol and the like, and the deposited precipitate is filtrated before drying, and the like, singly or in combination. When the purity of the polymer compound (A) is low, it can be purified by usual methods such as, for example, recrystallization, reprecipitation, continuous extraction with a Soxhlet extractor, column chromatography and the like.

### [Compound (B)]

The compound (B) is a compound having a condensed hetero ring skeleton (b) containing a boron atom and a nitrogen atom in the ring.

In the compound (B), it is preferable that at least one of nitrogen atoms contained in the condensed hetero ring skeleton (b) is a nitrogen atom not forming a double bond, and it is more preferable that all nitrogen atoms contained in the condensed hetero ring skeleton (b) are nitrogen atoms not forming a double bond.

The number of carbon atoms of the condensed hetero ring skeleton (b), not including the number of carbon atoms of the substituent, is usually 1 to 60, preferably 5 to 40, and more preferably 10 to 25.

The number of hetero atoms of the condensed hetero ring skeleton (b), not including the number of hetero atoms of the substituent, is usually 2 to 30, preferably 2 to 15, more preferably 2 to 10, further preferably 2 to 5, and particularly preferably 2 or 3.

The number of boron atoms of the condensed hetero ring skeleton (b), not including the number of boron atoms of the substituent, is usually 1 to 10, preferably 1 to 5, more preferably 1 to 3, and further preferably 1.

The number of nitrogen atoms of the condensed hetero ring skeleton (b), not including the number of nitrogen atoms of the substituent, is usually 1 to 20, preferably 1 to 10, more preferably 1 to 5, further preferably 1 to 3, and particularly preferably 2.

The condensed hetero ring skeleton (b) is preferably a tri to dodecacyclic condensed hetero ring skeleton, more preferably a tri to hexacyclic condensed hetero ring skeleton, and further preferably a pentacyclic condensed hetero ring skeleton, since the light emitting device of the present embodiment is more excellent in external quantum efficiency.

The compound (B) can also be referred to as a compound having a hetero ring group (b') containing a condensed hetero ring skeleton (b).

The hetero ring group (b') may be a group obtained by removing from a polycyclic heterocyclic compound containing a boron atom and a nitrogen atom in the ring one or more hydrogen atoms bonding directly to atoms constituting the ring, and the group optionally has a substituent.

The substituent which the hetero ring group (b') optionally has is preferably a halogen atom, a cyano group, an alkyl group, a cycloalkyl group, an alkoxy group, a cycloalkoxy group, an aryl group, a monovalent hetero ring group or a substituted amino group, more preferably an alkyl group, a cycloalkyl group, an alkoxy group, a cycloalkoxy group, an aryl group, a monovalent hetero ring group or a substituted amino group, and further preferably an alkyl group, an aryl group or a substituted amino group, and these groups optionally further have a substituent.

The aryl group as the substituent which the hetero ring group (b') optionally has is preferably a group obtained by removing from a monocyclic or dicyclic to hexacyclic aromatic hydrocarbon one hydrogen atom bonding directly to an atom constituting the ring, more preferably a group obtained by removing from a monocyclic, dicyclic or tricyclic aromatic hydrocarbon one hydrogen atom bonding directly to an atom constituting the ring, further preferably a group obtained by removing from benzene, naphthalene, anthracene, phenanthrene or fluorene one hydrogen atom bonding directly to an atom constituting the ring, and particularly preferably a phenyl group, and these groups optionally have a substituent.

The monovalent hetero ring group as the substituent which the hetero ring group (b') optionally has is preferably a group obtained by removing from a monocyclic or dicyclic to hexacyclic heterocyclic compound one hydrogen atom bonding directly to an atom constituting the ring, more preferably a group obtained by removing from a monocyclic, dicyclic or tricyclic heterocyclic compound one hydrogen atom bonding directly to an atom constituting the ring, further preferably a group obtained by removing from pyridine, diazabenzene, triazine, azanaphthalene, diazanaphthalene, carbazole, dibenzofuran, dibenzothiophene, phenoxazine or phenothiazine one hydrogen atom bonding directly to an atom constituting the ring, and particularly preferably a group obtained by removing from pyridine, diazabenzene or triazine one hydrogen atom bonding directly to an atom constituting the ring, and these groups optionally have a substituent.

In the substituted amino group as the substituent which the hetero ring group (b') optionally has, the substituent which the amino group has is preferably an aryl group or a monovalent hetero ring group, and more preferably an aryl group, and these groups optionally further have a substituent. The examples and preferable ranges of the aryl group and the monovalent hetero ring group as the substituent which the amino group has are the same as the examples and preferable ranges of the aryl group and the monovalent hetero ring group as the substituent which the hetero ring group (b') optionally has, respectively.

The substituent which the substituent which the hetero ring group (b') optionally has optionally further has is preferably a halogen atom, an alkyl group, a cycloalkyl group, an alkoxy group, a cycloalkoxy group, an aryl group, a monovalent hetero ring group or a substituted amino group, more preferably an alkyl group, a cycloalkyl group, an aryl group, a monovalent hetero ring group or a substituted amino group, and further preferably an alkyl group or a cycloalkyl group, and these groups optionally further have a substituent, but it is preferable that they do not further have a substituent.

The examples and preferable ranges of the aryl group, the monovalent hetero ring group and the substituted amino group as the substituent which the substituent which the hetero ring group (b') optionally has optionally further has are the same as the examples and preferable ranges of the aryl group, the monovalent hetero ring group and the substituted amino group as the substituent which the hetero ring group (b') optionally has, respectively.

"The nitrogen atom not forming a double bond" means a nitrogen atom that is single-bonded to each of the other three atoms.

The phrase "containing a nitrogen atom not forming a double bond in the ring" means that a group represented by -N(-R^{N})- (wherein, R^{N} represents a hydrogen atom or a substituent) or the formula: 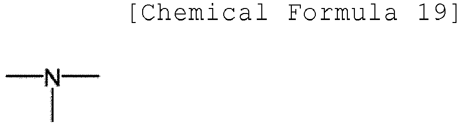 is contained in the ring.

The compound (B) is preferably a thermally activated delayed fluorescence (TADF) compound, since the light emitting device of the present embodiment is more excellent in external quantum efficiency.

ΔE_{ST} of the compound (B) may be 2.0 eV or less, may be 1.5 eV or less, may be 1.0 eV or less, may be 0.80 eV or less or may be 0.60 eV or less, and it is preferably 0.50 eV or less, since the light emitting device of the present embodiment is more excellent in external quantum efficiency. Meanwhile, ΔE_{ST} of the compound (B) may be 0.001 eV or more, may be 0.01 eV or more, may be 0.10 eV or more, may be 0.20 eV or more, may be 0.30 eV or more, or may be 0.40 eV or more.

The compound (B) is preferably a low molecular weight compound.

The molecular weight of the compound (B) is preferably 1×10² to 5×10³, more preferably 2×10² to 3×10³, further preferably 3×10² to 1.5×10³, and particularly preferably 4×10² to 1×10³.

The compound (B) is preferably a compound represented by the formula (1-1), the formula (1-2) or the formula (1-3), more preferably a compound represented by the formula (1-2) or the formula (1-3), and further preferably a compound represented by the formula (1-2), since the light emitting device of the present embodiment is more excellent in external quantum efficiency.

Ar¹, Ar² and Ar³ are each preferably a group obtained by removing from a monocyclic, dicyclic or tricyclic aromatic hydrocarbon or a monocyclic, dicyclic or tricyclic heterocyclic compound one or more hydrogen atoms bonding directly to atoms constituting the ring, more preferably a group obtained by removing from a monocyclic aromatic hydrocarbon or a monocyclic heterocyclic compound one or more hydrogen atoms bonding directly to atoms constituting the ring, further preferably a group obtained by removing from benzene, pyridine or diazabenzene one or more hydrogen atoms bonding directly to atoms constituting the ring, and particularly preferably a group obtained by removing from benzene one or more hydrogen atoms bonding directly to atoms constituting the ring, since the light emitting device of the present embodiment is more excellent in external quantum efficiency, and these groups optionally have a substituent.

The examples and preferable ranges of the substituent which Ar¹, Ar² and Ar³ optionally have are the same as the examples and preferable ranges of the substituent which the hetero ring group (b') optionally has.

Y² and Y³ are each preferably a single bond, an oxygen atom, a sulfur atom, a group represented by - N(Ry)- or a methylene group, more preferably a single bond, an oxygen atom, a sulfur atom or a group represented by -N(Ry)-, further preferably an oxygen atom, a sulfur atom or a group represented by -N(Ry)-, and particularly preferably a group represented by - N(Ry)-, and these groups optionally have a substituent.

The examples and preferable ranges of the substituent which Y¹, Y² and Y³ optionally have are the same as the examples and preferable ranges of the substituent which the hetero ring group (b') optionally has.

Ry is preferably an alkyl group, a cycloalkyl group, an aryl group or a monovalent hetero ring group, more preferably an aryl group or a monovalent hetero ring group, and further preferably an aryl group, and these groups optionally have a substituent.

The examples and preferable ranges of the aryl group and the monovalent hetero ring group represented by Ry are the same as the examples and preferable ranges of the aryl group and the monovalent hetero ring group as the substituent which the hetero ring group (b') optionally has, respectively.

The examples and preferable ranges of the substituent which the Ry optionally has are the same as the examples and preferable ranges of the substituent which the hetero ring group (b') optionally has.

Ry may be bonded directly or via a connecting group to Ar¹, Ar² or Ar³, but it is preferable that it is not bonded. The connecting group includes, for example, a group represented by -O-, a group represented by -S-, a group represented by -N(Ry)-, an alkylene group, a cycloalkylene group, an arylene group and a divalent hetero ring group, and is preferably a group represented by -O-, a group represented by -S-, a group represented by -N(Ry)- or a methylene group, and these groups optionally have a substituent.

As the compound (B), compounds represented by the following formulae and compounds B1 and B2 described later are exemplified.

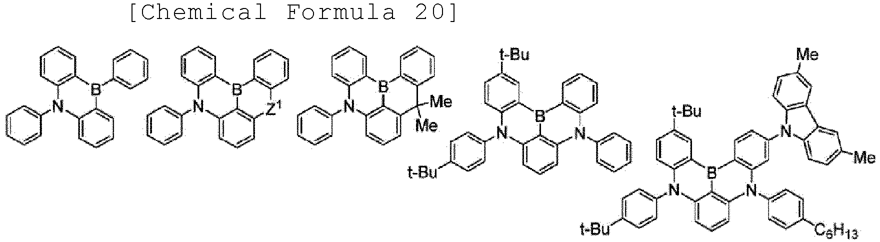

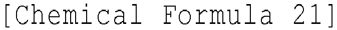

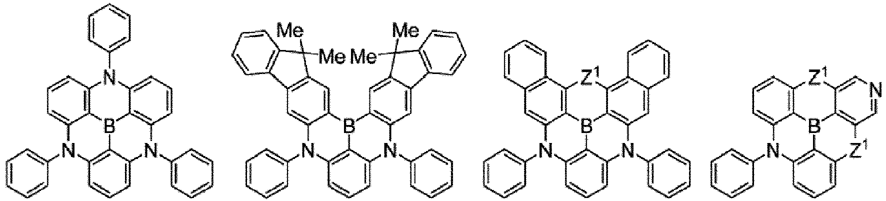

In the formulae, Z¹ represents an oxygen atom or a sulfur atom.

### [Other component]

The composition for light emitting device of the present embodiment may be a composition containing two or more of the compounds (A), the compound (B), and at least one selected from the group consisting of a hole transporting material, a hole injection material, an electron transporting material, an electron injection material, a light emitting material, an antioxidant and a solvent. The hole transporting material, the hole injection material, the electron transporting material, the electron injection material and the light emitting material are different from the compound (A) and the compound (B).

### [Ink]

The composition containing two or more of the compounds (A) and the compound (B), and a solvent (hereinafter, referred to as "ink") is suitable for fabricating a light emitting device using a wet method such as, for example, a spin coat method, a casting method, a microgravure coat method, a gravure coat method, a bar coat method, a roll coat method, a wire bar coat method, a dip coat method, a spray coat method, a screen printing method, a flexo printing method, an offset printing method, an inkjet printing method, a capillary coat method, a nozzle coat method and the like. The viscosity of the ink may be adjusted according to the type of the printing method, and is preferably 1 mPa•s to 20 mPa•s at 25°C.

The solvent contained in the ink is preferably a solvent capable of dissolving or uniformly dispersing solid components in the ink. The solvent includes, for example, chlorine-based solvents, ether-based solvents, aromatic hydrocarbon-based solvents, aliphatic hydrocarbon-based solvents, ketone-based solvents, ester-based solvents, polyhydric alcohol-based solvents, alcohol-based solvents, sulfoxide-based solvents and amide-based solvents.

In the ink, the compounding amount of the solvent is usually 1000 parts by mass to 100000 parts by mass, when the sum of the compound (A) and the compound (B) is taken as 100 parts by mass.

The solvent may be used singly or in combination of two or more.

### • Hole transporting material

The hole transporting material is classified into low molecular weight compounds and polymer compounds, and polymer compounds having a cross-linkable group are preferable.

The polymer compound includes, for example, polyvinylcarbazole and derivatives thereof; and polyarylenes having an aromatic amine structure in the side chain or main chain, and derivatives thereof. The polymer compound may be a compound to which an electron accepting site such as fullerene, tetrafluorotetracyanoquinodimethane, tetracyanoethylene, trinitrofluorenone and the like is bonded.

In the composition for light emitting device of the present embodiment, when a hole transporting material is contained, the compounding amount of the hole transporting material is usually 1 part by mass to 1000 parts by mass, when the sum of the compound (A) and the compound (B) is taken as 100 parts by mass.

The hole transporting material may be used singly or in combination of two or more.

### • Electron transporting material

The electron transporting material is classified into low molecular weight compounds and polymer compounds. The electron transporting material may have a cross-linkable group.

The low molecular weight compound includes, for example, a metal complex having 8-hydroxyquinoline as a ligand, oxadiazole, anthraquinodimethane, benzoquinone, naphthoquinone, anthraquinone, tetracyanoanthraquinodimethane, fluorenone, diphenyldicyanoethylene and diphenoquinone, and derivatives thereof.

The polymer compound includes, for example, polyphenylene, polyfluorene, and derivatives thereof. The polymer compound may be doped with a metal.

In the composition for light emitting device of the present embodiment, when an electron transporting material is contained, the compounding amount of the electron transporting material is usually 1 part by mass to 1000 parts by mass, when the sum of the compound (A) and the compound (B) is taken as 100 parts by mass.

The electron transporting material may be used singly or in combination of two or more.

### • Hole injection material and electron injection material

The hole injection material and the electron injection material are each classified into low molecular weight compounds and polymer compounds. The hole injection material and the electron injection material may have a cross-linkable group.

The low molecular weight compound includes, for example, metal phthalocyanines such as copper phthalocyanine and the like; carbon; oxides of metals such as molybdenum, tungsten and the like; metal fluorides such as lithium fluoride, sodium fluoride, cesium fluoride, potassium fluoride and the like.

The polymer compound includes electrically conductive polymers such as, for example, polyaniline, polythiophene, polypyrrole, polyphenylenevinylene, polythienylenevinylene, polyquinoline and polyquinoxaline, and derivatives thereof; a polymer containing an aromatic amine structure in the main chain or side chain, and the like.

In the composition for light emitting device of the present embodiment, when a hole injection material and/or an electron injection material is contained, the compounding amounts of the hole injection material and the electron injection material are each usually 1 part by mass to 1000 parts by mass, when the sum of the compound (A) and the compound (B) is taken as 100 parts by mass.

The hole injection material and the electron injection material each may be used singly or in combination of two or more.

### • Ion doping

When the hole injection material or the electron injection material contains an electrically conductive polymer, the electric conductivity of the electrically conductive polymer is preferably 1×10⁻⁵ S/cm to 1×10³ S/cm. For adjusting the electric conductivity of the electrically conductive polymer within such a range, the electrically conductive polymer can be doped with an appropriate amount of ions. The kind of the ion to be doped is an anion for the hole injection material and a cation for the electron injection material. The anion includes, for example, a polystyrenesulfonic ion, an alkylbenzenesulfonic ion and a camphorsulfonic ion. The cation includes, for example, a lithium ion, a sodium ion, a potassium ion and a tetrabutylammonium ion.

The ions to be doped may be used singly or in combination of two or more.

### • Light emitting material

The light emitting material is classified into low molecular weight compounds and polymer compounds. The light emitting material may have a cross-linkable group.

The low molecular weight compound includes, for example, naphthalene and derivatives thereof, anthracene and derivatives thereof, perylene and derivatives thereof, and triplet light emitting complexes having iridium, platinum or europium as the central metal.

The polymer compound includes polymer compounds containing, for example, an arylene group such as a phenylene group, a naphthalenediyl group, a fluorenediyl group, a phenanthrenediyl group, a dihydrophenanthrenediyl group, an anthracenediyl group, a pyrenediyl group and the like; an aromatic amine residue such as a group obtained by removing from an aromatic amine two hydrogen atoms, and the like; and a divalent hetero ring group such as a carbazolediyl group, a phenoxazinediyl group, a phenothiazinediyl group and the like.

In the composition for light emitting device of the present embodiment, when a light emitting material is contained, the content of the light emitting material is usually 1 part by mass to 1000 parts by mass, when the sum of the compound (A) and the compound (B) is taken as 100 parts by mass.

The light emitting material may be used singly or in combination of two or more.

### • Antioxidant

The antioxidant may a compound which is soluble in the same solvent as for the compound (A) and the compound (B) and does not inhibit light emission and charge transportation, and includes, for example, phenol type antioxidants and phosphorus-based antioxidants.

In the composition for light emitting device of the present embodiment, when an antioxidant is contained, the compounding amount of the antioxidant is usually 0.001 parts by mass to 10 parts by mass, when the sum of the compound (A) and the compound (B) is taken as 100 parts by mass.

The antioxidant may be used singly or in combination of two or more.

<Film>

The film of the present embodiment contains the composition for light emitting device described above. The film of the present embodiment is suitable as a light emitting layer in a light emitting device. The film of the present embodiment can be fabricated, for example, by a wet method using an ink. Further, the film of the present embodiment can be fabricated, for example, by a dry method such as a vacuum vapor deposition method and the like. The method for fabricating the film of the present embodiment by a dry method includes, for example, a method of vapor-depositing the above-described composition for light emitting device and a method of co-vapor-depositing the compound (A) and the compound (B).

The thickness of the film is usually 1 nm to 10 µm.

### <Light emitting device>

The light emitting device of the present embodiment contains the composition for light emitting device described above.

The light emitting device of the present embodiment may be one having, for example, an anode, a cathode, and an organic layer containing the above-described composition for light emitting device disposed between the anode and the cathode.

### [Layer constitution]

The layer containing the composition for light emitting device of the present embodiment is usually one or more layers selected from the group consisting of a light emitting layer, a hole transporting layer, a hole injection layer, an electron transporting layer and an electron injection layer, and preferably is a light emitting layer. These layers contain a light emitting material, a hole transporting material, a hole injection material, an electron transporting material and an electron injection material, respectively. These layers can be formed by the same method as for the fabrication of the film described above using a light emitting material, a hole transporting material, a hole injection material, an electron transporting material and an electron injection material, respectively.

The light emitting device has a light emitting layer between an anode and a cathode. The light emitting device of the present embodiment preferably has at least one of a hole injection layer and a hole transporting layer between an anode and a light emitting layer, from the standpoint of hole injectability and hole transportability, and preferably has at least one of an electron injection layer and an electron transporting layer between a cathode and a light emitting layer, from the standpoint of electron injectability and electron transportability.

The materials of a hole transporting layer, an electron transporting layer, a light emitting layer, a hole injection layer and an electron injection layer include the hole transporting material, the electron transporting material, the light emitting material, the hole injection material and the electron injection material and the like described above, respectively, in addition to the composition for light emitting device of the present embodiment.

When the material of a hole transporting layer, the material of an electron transporting layer and the material of a light emitting layer are soluble in a solvent used in forming layers adjacent to the hole transporting layer, the electron transporting layer and the light emitting layer, respectively, in fabricating a light emitting device, it is preferable that the material has a cross-linkable group for avoiding the material from being dissolved in the solvent. After forming each layer using the material having a cross-linkable group, the cross-linkable group can be crosslinked to insolubilize the layer.

The method for forming each layer such as a light emitting layer, a hole transporting layer, an electron transporting layer, a hole injection layer, an electron injection layer and the like in the light emitting device of the present invention includes, for example, dry methods such as a method of vacuum vapor-deposition from a powder and the like and wet methods such as a method by film formation from a solution or melted state and the like when a low molecular weight compound is used, and includes, for example, wet methods such as a method by film formation from a solution or melted state and the like when a polymer compound is used. The order, number and thickness of layers to be laminated are adjusted in consideration of, for example, light emission efficiency, driving voltage and luminance life.

### [Substrate/electrode]

The substrate of a light emitting device may be a substrate on which an electrode can be formed and which does not chemically change in forming an organic layer, and it is, for example, a substrate made of a material such as glass, plastic, silicon and the like. In the case of an opaque substrate, it is preferable that the electrode farthest from the substrate is transparent or semi-transparent.

The material of the anode includes, for example, electrically conductive metal oxides and semi-transparent metals, preferably includes indium oxide, zinc oxide, tin oxide; electrically conductive compounds such as indium-tin-oxide (ITO), indium-zinc-oxide and the like; argentine-palladium-copper (APC) complex; NESA, gold, platinum, silver and copper.

The material of the cathode includes, for example, metals such as lithium, sodium, potassium, rubidium, cesium, beryllium, magnesium, calcium, strontium, barium, aluminum, zinc, indium and the like; alloys composed of two or more of them; alloys composed of one or more of them and one or more of silver, copper, manganese, titanium, cobalt, nickel, tungsten and tin; and graphite and graphite intercalation compounds. The alloy includes, for example, a magnesium-silver alloy, a magnesium-indium alloy, a magnesium-aluminum alloy, an indium-silver alloy, a lithium-aluminum alloy, a lithium-magnesium alloy, a lithium-indium alloy and a calcium-aluminum alloy.

The anode and the cathode each may take a laminated structure composed of two or more layers.

### [Application]

The light emitting device of the present embodiment can be suitably used as a light source for backlight of a liquid crystal display device, a light source for illumination, organic EL illumination, a display device of computers, televisions, portable terminals and the like (for example, organic EL display and organic EL television).

Suitable embodiments of the present invention have been explained above, but the present invention is not limited to them.

### EXAMPLES

The present invention will be illustrated in detail by examples below, but the present invention is not limited to these examples.

In examples, the polystyrene-equivalent number-average molecular weight (Mn) and the polystyrene-equivalent weight-average molecular weight (Mw) of a polymer compound were determined by size exclusion chromatography (SEC) described below using tetrahydrofuran as the mobile phase.

A polymer compound to be measured was dissolved at a concentration of about 0.05% by mass in tetrahydrofuran, and 10 µL of the solution was injected into SEC. The mobile phase was flowed at a flow rate of 1.0 mL/min. As the column, PLgel MIXED-B (manufactured by Polymer Laboratories Ltd.) was used. As the detector, a UV-VIS detector (manufactured by Tosoh Corp., trade name: UV-8320GPC) was used.

For calculation of the value of ΔE_{ST} of a compound, the ground state of the compound was structurally optimized by density-functional approach at B3LYP level, and in this procedure, 6-31G* was used as the basis function. Using Gaussian09 as the quantum chemistry calculation program, ΔE_{ST} of the compound was calculated by time-dependent density-functional approach at B3LYP level.

In examples, the maximum peak wavelength of the light emission spectrum of a compound at room temperature was measured by a spectrophotometer (manufactured by JASCO Corporation, FP-6500) at room temperature. A compound was dissolved in xylene at a concentration of about 0.8×10⁻⁴% by mass and the resultant xylene solution was used as a specimen. As the excitation light, ultraviolet (UV) light having a wavelength of 325 nm was used.

### <Synthesis Example M1> Synthesis of compounds M1 to M6

A compound M1 was synthesized according to a method described in International Publication WO2015/145871.

A compound M2 was synthesized according to a method described in International Publication WO2013/146806.

A compound M3 was synthesized according to a method described in International Publication WO2005/049546.

A compound M4 was synthesized according to a method described in Japanese Unexamined Patent Application Publication (JP-A) No. 2010-189630.

A compound M5 and a compound M6 were synthesized according to a method described in International Publication WO2013/191088.

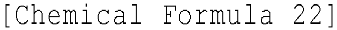

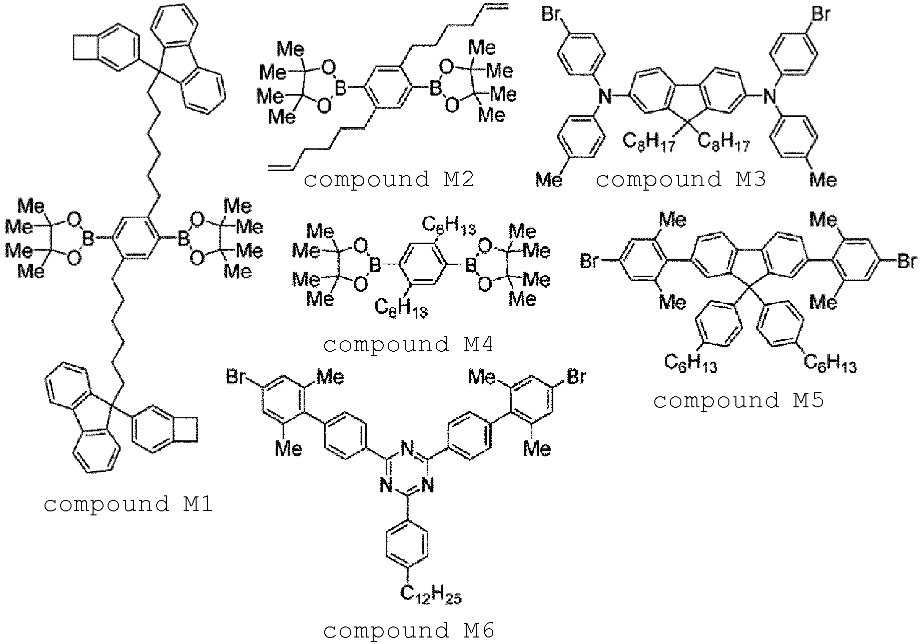

### <Synthesis Example HTL-1> Synthesis of polymer compound HTL-1

A polymer compound HTL-1 was synthesized according to a method described in International Publication WO2015/145871 using the compound M1, the compound M2 and the compound M3. The polymer compound HTL-1 had an Mn of 2.3×10⁴ and an Mw of 1.2×10⁵.

The polymer compound HTL-1 is a copolymer having a constitutional unit derived from the compound M1, a constitutional unit derived from the compound M2 and a constitutional unit derived from the compound M3 at a molar ratio of 45:5:50, according to the theoretical values calculated from the amounts of the charged raw materials.

### <Synthesis Example HP1>Synthesis of polymer compound HP-1

A polymer compound HP-1 was synthesized according to a method described in International Publication WO2015/008851 using the compound M4, the compound M5 and the compound M6. The polymer compound HP-2 had an Mn of 8.5×10⁴ and an Mw of 2.2×10⁵.

The polymer compound HP-1 is a copolymer having a constitutional unit derived from the compound M4, a constitutional unit derived from the compound M5 and a constitutional unit derived from the compound M6 at a molar ratio of 50:26:24, according to the theoretical values calculated from the amounts of the charged raw materials.

### <Acquisition and synthesis of compounds H1 to H8, B1 to B2 and polymer compound PVK>

A compound H1 to a compound H7 manufactured by Luminescence Technology Corp. were used.

A compound H8 was synthesized according to a method described in JP-A No. 2010-031259.

Poly(9-vinylcarbazole) (polymer compound PVK) manufactured by Sigma-Aldrich (weight-average molecular weight: 1.1×10⁶, powdery) was used.

A compound B1 and a compound B2 were synthesized with reference to a method described in International Publication WO2015/102118.

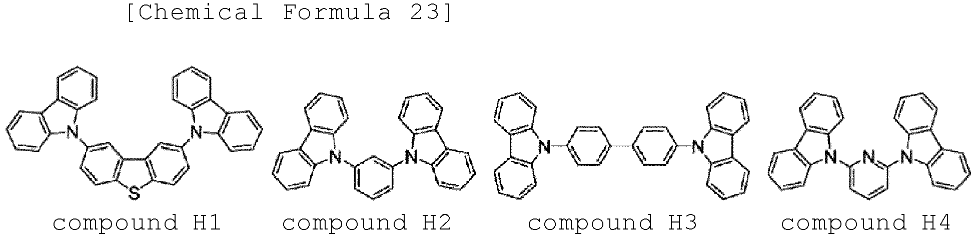

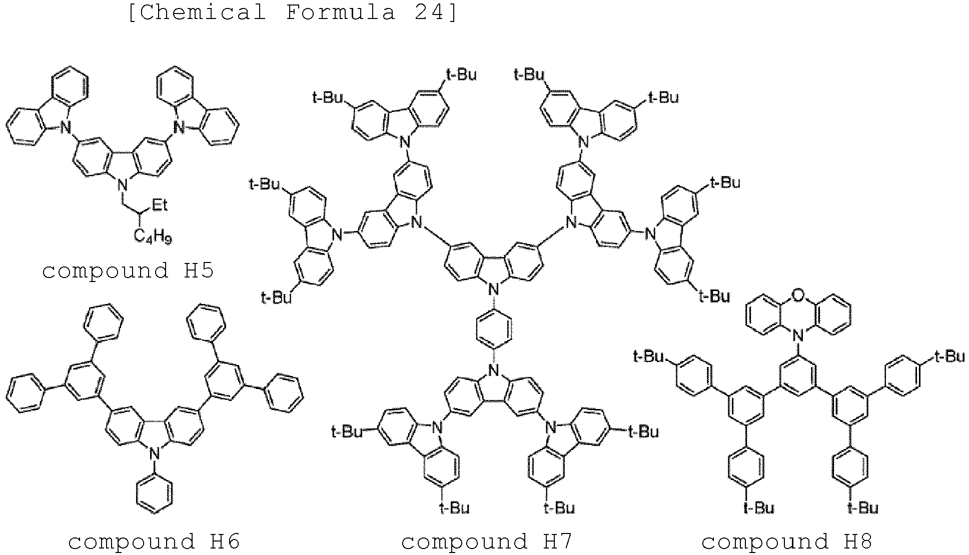

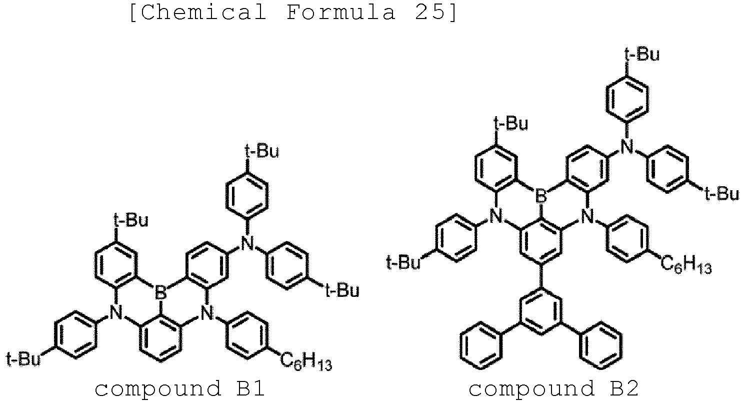

The maximum peak wavelength of the emission spectrum at room temperature of the compound B1 was 453 nm. The half-value width of the maximum peak of the emission spectrum at room temperature of the compound B1 was 22 nm. ΔE_{ST} of the compound B1 was 0.457 eV.

The maximum peak wavelength of the emission spectrum at room temperature of the compound B2 was 467 nm. The half-value width of the maximum peak of the emission spectrum at room temperature of the compound B2 was 20 nm. ΔE_{ST} of the compound B2 was 0.457 eV.

### <Example D1> Fabrication and evaluation of light emitting device D1

### (Formation of anode and hole injection layer)

An ITO film was attached with a thickness of 45 nm to a glass substrate by a sputtering method, to form an anode. On the anode, a hole injection material ND-3202 (manufactured by Nissan Chemical Corp.) was spin-coated, to form a film with a thickness of 35 nm. The substrate carrying the hole injection layer laminated thereon was heated on a hot plate at 50°C for 3 minutes under an air atmosphere, and further heated at 230°C for 15 minutes, to form a hole injection layer.

### (Formation of hole transporting layer)

The polymer compound HTL-1 was dissolved in xylene at a concentration of 0.7% by mass. The resultant xylene solution was spin-coated on the hole injection layer, to form a film with a thickness of 20 nm, and the film was heated on a hot plate at 180°C for 60 minutes under a nitrogen gas atmosphere, to form a hole transporting layer.

### (Formation of light emitting layer)

The polymer compound HP-1, the compound H2 and the compound B1 (polymer compound HP-1/compound H2/compound B1 = 94% by mass/5% by mass/1% by mass) were dissolved at a concentration of 2% by mass in toluene. The resultant toluene solution was spin-coated on the hole transporting layer, to form a film with a thickness of 60 nm, and the film was heated at 130°C for 10 minutes under a nitrogen gas atmosphere, to form a light emitting layer.

### (Formation of cathode)

The substrate carrying the light emitting layer formed thereon was place in a vapor deposition machine, and the inner pressure thereof was reduced to 1.0×10⁻⁴ Pa or less, then, as the cathode, sodium fluoride was vapor-deposited with a thickness of 4 nm on the light emitting layer, then, aluminum was vapor-deposited with a thickness of about 80 nm on the sodium fluoride layer. After vapor deposition, the substrate carrying the cathode formed thereon was sealed with a glass substrate, to fabricate a light emitting device D1.

### (Evaluation of light emitting device)

Voltage was applied to the light emitting device D1, to observe EL light emission. The external quantum efficiency [%] at 0.2 mA/cm² was measured.

### <Example D2 to D16 and Comparative Example CD1>

### Fabrication and evaluation of light emitting devices D2 to D16 and CD1

Light emitting devices D2 to D16 and CD1 were fabricated in the same manner as in Example D1, except that materials described in Table 2 were used instead of "the polymer compound HP-1, the compound H2 and the compound B1 (polymer compound HP-1/compound H2/compound B1 = 94% by mass/5% by mass/1% by mass)" in (Formation of light emitting layer) of Example D1.

Voltage was applied to the light emitting devices D2 to D16 and CD1, to observe EL light emission. The external quantum efficiency [%] at 0.2 mA/cm² was measured.

The results of Examples D1 to D16 and Comparative Example CD1 are shown in Table 2. The relative values of the external quantum efficiency of the light emitting devices D1 to D16, when the external quantum efficiency of the light emitting device CD1 is taken as 1.0, are shown.

**[Table 2]**

| | Light emitting device | Light emitting layer | | External quantum efficiency (relative value) |
|---|---|---|---|---|
| | | Material | Composition ratio (% by mass) | |
| Example D1 | D1 | HP-1/H2/B1 | 94/5/1 | 2.3 |
| Example D2 | D2 | HP-1/H3/B1 | 94/5/1 | 1.7 |
| Example D3 | D3 | HP-1/H4/B1 | 94/5/1 | 2.2 |
| Example D4 | D4 | HP-1/H5/B1 | 94/5/1 | 1.7 |
| Example D5 | D5 | HP-1/H7/B1 | 94/5/1 | 1.6 |
| Example D6 | D6 | HP-1/H8/B1 | 94/5/1 | 1.6 |
| Example D7 | D7 | HP-1/PVK/B1 | 94/5/1 | 1.6 |
| Example D8 | D8 | HP-1/H1/B2 | 94/5/1 | 1.6 |
| Example D9 | D9 | HP-1/H2/B2 | 94/5/1 | 1.8 |
| Example D10 | D10 | HP-1/H3/B2 | 94/5/1 | 1.8 |
| Example D11 | D11 | HP-1/H4/B2 | 94/5/1 | 2.0 |
| Example D12 | D12 | HP-1/H5/B2 | 94/5/1 | 2.0 |
| Example D13 | D14 | HP-1/H6/B2 | 94/5/1 | 1.5 |
| Example D14 | D13 | HP-1/H7/B2 | 94/5/1 | 1.4 |
| Example D15 | D15 | HP-1/H8/B2 | 94/5/1 | 1.6 |
| Example D16 | D16 | HP-1/PVK/B2 | 94/5/1 | 1.3 |
| Comparative Example CD1 | CD1 | HP-1/B1 | 99/1 | 1.0 |

### Industrial Applicability

The composition of the present invention is useful for producing a light emitting device excellent in external quantum efficiency.

## Claims

1. A light emitting device comprising
an anode,
a cathode, and
an organic layer disposed between said anode and said cathode and containing a composition for light emitting device, wherein
said composition for light emitting device contains
at least two compounds (A) selected from the group consisting of a compound represented by the formula (FH) and a polymer compound containing a constitutional unit having a group obtained by removing from a compound represented by the formula (FH) one or more hydrogen atoms, and
a compound (B) having a condensed hetero ring skeleton (b) containing a boron atom and a nitrogen atom in the ring: 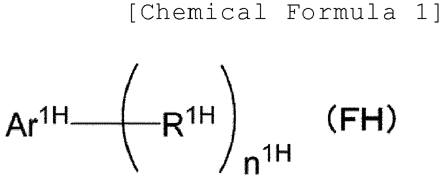 wherein,
n^{1H} represents an integer of 0 or more,
Ar^{1H} represents a group obtained by removing from a heterocyclic compound containing a nitrogen atom in the ring n^{1H} or more hydrogen atoms bonding directly to atoms constituting the ring, and this group optionally has a substituent, when a plurality of the substituents are present, they may be the same or different and may be combined together to form a ring together with atoms to which they are attached, the above-described heterocyclic compound is a heterocyclic compound not containing said condensed hetero ring skeleton (b),
R^{1H} represents an aryl group, a monovalent hetero ring group or a substituted amino group, and these groups optionally have a substituent, when a plurality of the substituents are present, they may be the same or different and may be combined together to form a ring together with atoms to which they are attached, when a plurality of R^{1H} are present, they may be the same or different and may be combined together to form a ring together with atoms to which they are attached, the above-described monovalent hetero ring group is a group obtained by removing from a heterocyclic compound not containing said condensed hetero ring skeleton (b) one hydrogen atom bonding directly to an atom constituting the ring, and this group optionally has a substituent,
the substituent which Ar^{1H} optionally has and R^{1H} may be combined together to form a ring together with atoms to which they are attached.

2. The light emitting device according to Claim 1, wherein at least one of said polymer compound is contained as said compound (A).

3. The light emitting device according to Claim 1 or 2, wherein said heterocyclic compound in said Ar^{1H} is a monocyclic heterocyclic compound containing a nitrogen atom in the ring, a dicyclic heterocyclic compound containing a nitrogen atom in the ring or a tricyclic heterocyclic compound containing a nitrogen atom in the ring.

4. The light emitting device according to Claim 3, wherein said heterocyclic compound in said Ar^{1H} is oxadiazole, thiadiazole, pyrrole, diazole, triazole, pyridine, diazabenzene, triazine, azanaphthalene, diazanaphthalene, indole, benzodiazole, carbazole, azacarbazole, diazacarbazole, phenoxazine, phenothiazine, 9,10-dihydroacridine, 5,10-dihydrophenazine, azaanthracene, diazaanthracene, azaphenanthrene or diazaphenanthrene.

5. The light emitting device according to any one of Claims 1 to 4, wherein said compound (B) is a compound represented by the formula (1-1), a compound represented by the formula (1-2) or a compound represented by the formula (1-3): 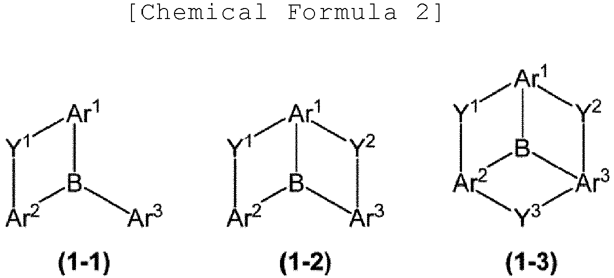 wherein,
Ar¹, Ar² and Ar³ each independently represent an aromatic hydrocarbon group or a hetero ring group, and these groups optionally have a substituent, when a plurality of the substituents are present, they may be the same or different and may be combined together to form a ring together with atoms to which they are attached,
Y¹ represents a group represented by -N(Ry)-,
Y² and Y³ each independently represent a single bond, an oxygen atom, a sulfur atom, a selenium atom, a group represented by -N(Ry)-, an alkylene group or a cycloalkylene group, and these groups optionally have a substituent, when a plurality of the substituents are present, they may be the same or different and may be combined together to form a ring together with atoms to which they are attached, Ry represents a hydrogen atom, an alkyl group, a cycloalkyl group, an aryl group or a monovalent hetero ring group, and these groups optionally have a substituent, when a plurality of the substituents are present, they may be the same or different and may be combined together to form a ring together with atoms to which they are attached, when a plurality of Ry are present, they may be the same or different, Ry may be bonded directly or via a connecting group to Ar¹, Ar² or Ar³.

6. The light emitting device according to Claim 5, wherein said Y² and said Y³ are each a group represented by -N(Ry)-.

7. The light emitting device according to any one of Claims 1 to 6, wherein said composition for light emitting device further contains at least one selected from the group consisting of a hole transporting material, a hole injection material, an electron transporting material, an electron injection material, a light emitting material, an antioxidant and a solvent.

8. A composition for light emitting device comprising
at least two compounds (A) selected from the group consisting of a compound represented by the formula (FH) and a polymer compound containing a constitutional unit having a group obtained by removing from a compound represented by the formula (FH) one or more hydrogen atoms, and
a compound (B) having a condensed hetero ring skeleton (b) containing a boron atom and a nitrogen atom in the ring: 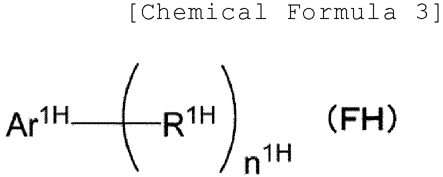 wherein,
n^{1H} represents an integer of 0 or more,
Ar^{1H} represents a group obtained by removing from a heterocyclic compound containing a nitrogen atom in the ring n^{1H} or more hydrogen atoms bonding directly to atoms constituting the ring, and this group optionally has a substituent, when a plurality of the substituents are present, they may be the same or different and may be combined together to form a ring together with atoms to which they are attached, the above-described heterocyclic compound is a heterocyclic compound not containing said condensed hetero ring skeleton (b),
R^{1H} represents an aryl group, a monovalent hetero ring group or a substituted amino group, and these groups optionally have a substituent, when a plurality of the substituents are present, they may be the same or different and may be combined together to form a ring together with atoms to which they are attached, when a plurality of R^{1H} are present, they may be the same or different and may be combined together to form a ring together with atoms to which they are attached, the above-described monovalent hetero ring group is a group obtained by removing from a heterocyclic compound not containing said condensed hetero ring skeleton (b) one hydrogen atom bonding directly to an atom constituting the ring, and this group optionally has a substituent,
the substituent which Ar^{1H} optionally has and R^{1H} may be combined together to form a ring together with atoms to which they are attached.

9. The composition for light emitting device according to Claim 8, wherein at least one of said polymer compound is contained as said compound (A).

10. The composition for light emitting device according to Claim 8 or 9, wherein said heterocyclic compound in said Ar^{1H} is a monocyclic heterocyclic compound containing a nitrogen atom in the ring, a dicyclic heterocyclic compound containing a nitrogen atom in the ring or a tricyclic heterocyclic compound containing a nitrogen atom in the ring.

11. The composition for light emitting device according to any one of Claims 8 to 10, wherein said compound (B) is a compound represented by the formula (1-1), a compound represented by the formula (1-2) or a compound represented by the formula (1-3): 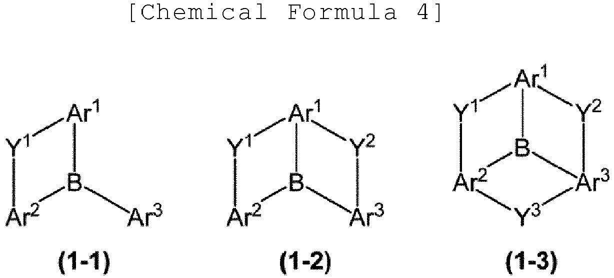 wherein,
Ar¹, Ar² and Ar³ each independently represent an aromatic hydrocarbon group or a hetero ring group, and these groups optionally have a substituent, when a plurality of the substituents are present, they may be the same or different and may be combined together to form a ring together with atoms to which they are attached,
Y¹ represents a group represented by -N(Ry)-,
Y² and Y³ each independently represent a single bond, an oxygen atom, a sulfur atom, a selenium atom, a group represented by -N(Ry)-, an alkylene group or a cycloalkylene group, and these groups optionally have a substituent, when a plurality of the substituents are present, they may be the same or different and may be combined together to form a ring together with atoms to which they are attached, Ry represents a hydrogen atom, an alkyl group, a cycloalkyl group, an aryl group or a monovalent hetero ring group, and these groups optionally have a substituent, when a plurality of the substituents are present, they may be the same or different and may be combined together to form a ring together with atoms to which they are attached, when a plurality of Ry are present, they may be the same or different, Ry may be bonded directly or via a connecting group to Ar¹, Ar² or Ar³.

12. The composition for light emitting device according to Claim 11, wherein said Y² and said Y³ are each a group represented by -N(Ry)-.

13. The composition for light emitting device according to any one of Claims 8 to 12, further comprising at least one selected from the group consisting of a hole transporting material, a hole injection material, an electron transporting material, an electron injection material, a light emitting material, an antioxidant and a solvent.
